# EUROPÄISCHE PATENTSCHRIFT

(11) **EP 2 673 747 B1**
(45) Veröffentlichungstag und Bekanntmachung des Hinweises auf die Patenterteilung: **08.03.2017**
(21) Anmeldenummer: 11794651.7
(22) Anmeldetag: 12.12.2011
(51) Int. Cl.: G06T 7/00, A61C 13/00

(54) **VERFAHREN UND ANALYSESYSTEM ZUR GEOMETRISCHEN ANALYSE VON SCANDATEN ORALER STRUKTUREN**
METHOD AND ANALYSIS SYSTEM FOR THE GEOMETRIC ANALYSIS OF SCAN DATA FROM ORAL STRUCTURES
PROCÉDÉ ET SYSTÈME D'ANALYSE POUR L'ANALYSE GÉOMÉTRIQUE DE DONNÉES DE BALAYAGE DE STRUCTURES ORALES

(30) Priorität: 10.02.2011 DE 102011010975
(43) Veröffentlichungstag der Anmeldung: 18.12.2013
(73) Patentinhaber: Straumann Holding AG, 4002 Basel (CH)
(72) Erfinder: TANK, Martin, 69115 Heidelberg (DE)
(74) Vertreter: Beckord & Niedlich
(86) Internationale Anmeldenummer: PCT/EP2011/006256
(87) Internationale Veröffentlichungsnummer: WO 2012/107069

(56) Entgegenhaltungen:
- WO-A2-2004/044787
- DE-A1-102007 033 998
- DE-C1- 19 642 247
- US-B2- 7 040 896
- BLANZ V ET AL: "A statistical method for robust 3D surface reconstruction from sparse data", 3D DATA PROCESSING, VISUALIZATION AND TRANSMISSION, 2004. 3DPVT 2004. PROCEEDINGS. 2ND INTERNATIONAL SYMPOSIUM ON THESSALONIKI, GREECE 6-9 SEPT. 2004, PISCATAWAY, NJ, USA,IEEE, 6. September 2004 (2004-09-06), Seiten 293-300, XP010725119, DOI: 10.1109/TDPVT.2004.1335212 ISBN: 978-0-7695-2223-4

## Beschreibung

Die Erfindung betrifft ein Verfahren zur geometrischen Analyse von Scandaten oraler Strukturen, welches vor allem im Rahmen der digitalen dentalen Technologie eingesetzt werden kann. Die ermittelten Informationen sind von grundlegender Bedeutung für die Herstellung von Zahnersatzteilen oder Zahnrestaurationsteilen durch dentale CAD/CAM-Systeme. Solche Systeme sind mittlerweile in der Zahnmedizin und Zahntechnik fest etabliert, wobei man in den exemplarischen Patentschriften DE 10 2007 033998 A1, WO 2004/044787 A2, US 5217375A, US 7708560

B2, US 7581953 B2, EP 06 34 150 A1, EP 09 13 130 A2, DE 10 2005 033 738 A1 und WO 0239056 A1 Beschreibungen solcher Systeme findet. Aber auch auf anderen Gebieten der digitalen dentalen Technologie ist eine geometrische Analyse von Scandaten oraler Strukturen von grundlegender Bedeutung. Die Ergebnisse können beispielsweise auf dem Gebiet der Implantologie oder Kieferorthopädie sinnvoll verwendet werden, aber auch zur Optimierung des Messvorgangs oraler Strukturen, indem die Analyse der Scandaten während der Messung stattfindet und die Ergebnisse den Messvorgang beeinflussen. Unter oralen Strukturen werden im Rahmen der vorliegenden Erfindung Zähne mit ihren angrenzenden anatomischen Strukturen wie Zahnfleisch, Kieferknochen, Nervenbahnen etc. aber auch nichtnatürliche Strukturen wie Implantate, Abutments, sonstige Verankerungssysteme, Brackets, statische sowie funktionelle Bissregistrate etc. verstanden. Außerdem betrifft die Erfindung ein Verfahren zur Erzeugung einer Modelldatenbank zur Verwendung in einem derartigen Verfahren sowie ein Analysesystem zur geometrischen Analyse von Scandaten oraler Strukturen.

Auf dem Gebiet der digitalen dentalen Technologie werden Scandaten oraler Strukturen vor allem optisch oder radiologisch bestimmt. Weit verbreitet und kostengünstig sind optische Scanner, welche intraoral Oberflächenstrukturen direkt oder extraoral Abformungen von oralen Oberflächenstrukturen dreidimensional vermessen. Man erhält in der Regel triangulierte Oberflächendaten, die bei offenen Systemen im STL-Format abgespeichert werden können. Mittels radiologischer Scanner wie Computertomographen (CT) oder Digitalen Volumentomographen (DVT) werden Volumendatensätze oraler Strukturen durch Verwendung von Röntgenstrahlung erzeugt. Als allgemein akzeptiertes Dateiformat hat sich hier das DICOM-Format aus der medizinischen Bildgebung durchgesetzt.

Für eine Optimierung der Messung und/oder zur weiteren Planung der Untersuchung und/oder zur Diagnoseerstellung und/oder zur Behandlungsplanung müssen diese Scandaten in vielen Fällen bereits während der Untersuchung oder unmittelbar nach der Untersuchung geometrisch analysiert werden. Eine wesentliche Rolle bei der Analyse von Scandaten spielt die sogenannte "Segmentierung" von geometrischen Strukturen. Bei einer solchen Segmentierung werden die Scandaten des Untersuchungsobjektes so zerlegt, dass bestimmte Teilobjekte eines Untersuchungsobjekts, d.h. bestimmte geometrische Strukturen, die im Focus der jeweiligen Untersuchung stehen, von den übrigen Scandaten separiert werden. Ein anschauliches Beispiel hierfür ist die Separation von Zähnen untereinander und vom Zahnfleisch bzw. Kieferknochen, sowie die Zuordnung von Zahnnummern zu den separierten Zähnen. Es können aber auch nichtnatürliche Strukturen, wie Implantate, Brackets etc. durch eine Segmentierung von den übrigen Scandaten separiert werden.

Auf dem Gebiet der Prothetik werden aufgrund der nötigen hohen Präzision bei der Ermittlung bzw. Berechnung von virtuellen Zahnrestaurationen in der Regel optische Scandaten verwendet. Unter Zahnrestaurationsteilen werden im Rahmen der vorliegenden Erfindung jegliche Art von herstellbaren Objekten zur Versorgung von Zahndefekten zusammengefasst. Beispielhaft seien hier Inlays, Onlays, Teilkronen, Kronen, Teleskopkronen, Brücken, Veneers, Implantataufbauten, Teilprothesen und Prothesen genannt. Es sei hier nochmals betont, dass im Rahmen der vorliegenden Erfindung auch Zahnersatzteile der Einfachheit halber unter dem Begriff Zahnrestaurationsteile fallen. Unter dem Begriff der "virtuellen Zahnrestauration" (im Folgenden auch kürzer als "Zahnrestauration" bezeichnet) sind entsprechende elektronische Zahnrestaurationsdarstellungen, d.h. digitale dreidimensionale Repräsentierungen solcher Zahnrestaurationsteile zu verstehen. Zur Herstellung eines Zahnrestaurationsteils wird dann beispielsweise ein CAD/CAM-Datensatz der dazugehörenden virtuellen Zahnrestauration an eine Herstellungsmaschine übermittelt.

Bei der Versorgung von Zahndefekten findet in der Regel eine Präparation der Zähne statt, d.h. es werden Karies, altes Füllungsmaterial oder defekte Zahnanteile entfernt. Übrig bleibt für jeden Zahn die Restzahnsubstanz, deren Oberfläche sich in einen präparierten (Kavität) und unpräparierten Anteil aufteilt. Die Grenzlinie von unpräparierter Zahnoberfläche zu präparierter Zahnoberfläche wird dabei als Präparationslinie bezeichnet. Ergänzend zu den nur bei präparierten Zähnen vorhandenen Präparationslinien besitzt jeder Zahn zumindest eine Segmentierungslinie, welche einen Zahn von extradentalen Strukturen wie beispielsweise dem Zahnfleisch und/oder dem Kieferknochen und/oder den Nachbarzähnen trennt. D.h. eine Segmentierungslinie ist ein Beispiel für eine weitere Grenzlinie. Aus diesen Definitionen ergibt sich, dass die Segmentierungs- und/oder Präparationslinien unpräparierte Zahnoberflächen begrenzen.

Auch auf dem Gebiet der Kieferorthopädie ist die Kenntnis von Segmentierungslinien von grundlegender Bedeutung. Durch diese Grenzlinien lassen sich die Zähne vom Zahnfleisch und den Nachbarzähnen separieren und ermöglichen so auch eine kieferorthopädische Behandlungsplanung, bei der beispielsweise die separierten Zähne virtuell optimal aufgestellt werden. Für solch eine optimale Zahnaufstellung ist auch die Kenntnis von anatomischen Landmarken (Schneidekanten, Zahnhöcker, Fissuren, etc.) und Zahnachsen der Zähne wichtig.

Im Rahmen der vorliegenden Erfindung kann der Begriff "geometrische Analyse" von Scandaten die Segmentierung oraler Strukturen, die Bestimmung von Segmentierungs- und Präparationslinien aber auch die Bestimmung von anatomischen Landmarken, Zahnachsen, Richtungsbezeichnungen, Oberflächenregionen und weiteren charakteristischen geometrischen Strukturen umfassen. Dabei kann eine geometrische Analyse von radiologischen Scandaten auch die Segmentierung des Ober-, bzw. Unterkiefers mit den Verläufen der zu den Zähnen gehörenden Nerven umfassen. Diese Analyse ist insbesondere für die Planung von Zahnimplantaten wichtig. Im weiteren Sinn umfasst eine geometrische Analyse von Scandaten auch die Anpassung von geometrisch deformierbaren Zahnmodellen an die Scandaten. Dieser Anpassungsprozess kann als vorbereitende Maßnahme für die eigentliche geometrische Analyse angesehen werden.

Aufgrund der komplizierten Struktur der Scandaten ist die interaktive geometrische Analyse mit Hilfe einer grafischen Benutzeroberfläche oft schwierig durchzuführen und mit einem hohen Zeitaufwand verbunden. Eine Verbesserung stellt die Anwendung von modellbasierten Verfahren dar, bei denen morphologisches Vorwissen über die Zähne, evtl. mit ihren angrenzenden anatomischen Strukturen, in die geometrische Analyse einfließt.

Vor allem im medizinischen Bereich werden bereits Verfahren beschrieben, die eine modellbasierte Segmentierung von Scandaten ermöglichen. So beschreibt DE 103 57 206 B4 ein spezielles Segmentierungsverfahren für anatomische Strukturen des Menschen. Solche Ansätze aus dem medizinischen Bereich können aber nicht einfach auf die besondere Situation in der Zahnmedizin übertragen werden. Dort sind häufig Zähne präpariert, d.h. es sind nur Teile der zu detektierenden Zähne vorhanden und die Scandaten werden dann, aufgrund der nötigen hohen Präzision, in der Regel optisch erzeugt. Bei den so gemessenen Daten ist insbesondere auch die präzise Detektion von Segmentierungs-und Präparationslinien wichtig.

In der DE 10 2007 033998 wird ein Kronen-Modell beschrieben, das bereits aus gescannten Daten erstellt wurde, welches an das generische Zahnmodell (eine Art "Restzahnmodell", also ein Zahn ohne Krone) angepasst wird, um ein Gesamtmodell (bestehend aus Restzahn und Krone) zu erzeugen. Es werden also zwei zueinander kompatible Objekte, Restzahn und Krone, aneinander angepasst, jedoch nicht die Scandaten auf Basis des individualisierten Zahnmodells erst segmentiert oder eine Grenzlinie ermittelt. Ebenso wird hier auch die Gingival-Kurve bei der Anpassung des Zahnmodells als bereits bekannt vorausgesetzt, bzw. durch herkömmliche Verfahren ermittelt.

Ziel der WO 2004/044787 ist eine parametrisierte Modellierung von Zahnrestaurationen; nicht von Grenzlinien (Präparation- und Segmentierungslinien).

Es ist eine Aufgabe der vorliegenden Erfindung, ein Verfahren und Analysesystem zur einfachen und sicheren geometrischen Analyse von Scandaten oraler Strukturen zu beschreiben, welche die Analyse mit möglichst geringer Bediener-Interaktion und geringem Zeitaufwand zufriedenstellend erlaubt.

Diese Aufgabe wird durch ein Verfahren gemäß dem Patentanspruch 1 sowie durch ein Analysesystem gemäß Patentanspruch 15 gelöst.

Im Rahmen der vorliegenden Erfindung werden die grundlegenden Begriffe Zahntyp, Zahnmodell und Präparationstyp sehr allgemein verstanden. Ein Zahntyp wird im Rahmen der Erfindung als eine sehr allgemeine Gruppierungsmöglichkeit von Zähnen definiert. Beispielsweise kann eine Gruppierung nach der Zahnnummer und/oder dem Alter und/oder der Abrasion und/oder der Volkszugehörigkeit und/oder dem Geschlecht und/oder nach morphologischen Besonderheiten (Wurzelanzahl, Höckeranzahl, etc.) erfolgen. Die Gruppierung kann aber auch durch die Zugehörigkeit zu den Frontzähnen, Eckzähnen, Prämolaren, Molaren oder zum Ober- bzw. Unterkiefer durchgeführt werden. Möglich ist auch eine Gruppierung von Zähnen unterschiedlicher Zahnnummern zu einem komplexen Zahntyp. An dieser Stelle sei betont, dass in der Praxis die Gruppierung über die Zahnnummer bevorzugt ist, d.h. Zahntyp und Zahnnummer können dann synonym verwendet werden.

Ebenso sehr allgemein wird der Begriff Zahnmodell verwendet. Ein Zahnmodell kann dabei aus mehreren Teilmodellen bestehen, die beispielsweise anatomische Strukturen wie Zahnoberflächen, Zahnfleisch, Kieferknochen, Nervenstrukturen, etc., aber auch nichtnatürliche Strukturen wie Implantate, Abutments, sonstige Verankerungssysteme, Brackets, etc. beschreiben. Darüber hinaus kann ein Zahnmodell auch Grenzlinien, anatomische Landmarken, Zahnachsen, Richtungsbezeichnungen, Oberflächenregionen und weitere charakteristische geometrische Strukturen enthalten. Wichtig ist, dass zumindest diejenigen Anteile der Zähne modelliert werden, welche zur geometrischen Analyse von Scandaten nötig sind. Die anderen Teilmodelle dienen vorzugsweise vor allem der Stabilisierung des Individualisierungsvorgangs, da diese an dazugehörende Strukturen in den Scandaten angepasst werden können. Die wichtigsten Grenzlinien sind dabei die schon beschriebenen Segmentierungs- und Präparationslinien. Es können aber auch weitere Grenzlinien verwendet werden. Beispielsweise Separationslinien, die Anpassungsbereiche von modellierten nichtnatürlichen Strukturen untereinander trennen. Eine Verwendung solcher Linien bietet sich beispielsweise bei der Detektion von Implantaten in den Scandaten an. Ein Implantat-Teilmodell kann beispielsweise durch eine Separationslinie in einen aktiven Anpassungsbereich außerhalb des Kieferknochens und einen inaktiven Anpassungsbereich innerhalb des Kieferknochens getrennt werden. Solch ein Implantat-Teilmodell kann dann bei der geometrischen Analyse der Scandaten als Teilmodell eines Zahnmodells Verwendung finden.

Im Rahmen der vorliegenden Erfindung wird auch der Begriff Präparationstyp sehr allgemein verstanden. Neben den Präparationstypen Inlay, Onlay, Teilkrone, Krone, Brücke und Veneer kann auch der Begriff "Zahn" als Präparationstyp verwendet werden. Der "Zahn"-Präparationstyp dient der Beschreibung einer Situation, bei der keine Präparation vorliegt. Dieses Vorgehen ist insbesondere sinnvoll, wenn man zu jedem Zahn in einem Scandatensatz den Präparationstyp angeben soll. Beispielsweise können in einem Scandatensatz neben einer Inlaypräparation und einer Kronenpräparation mehrere unpräparierte Zähne enthalten sein. Diese können dann durch den Präparationstyp "Zahn" beschrieben werden. Ein vor allem im Rahmen der Implantologie wichtiger Präparationstyp ist der Präparationstyp Implantat. Er kann vorzugsweise bei Situationen Verwendung finden, bei denen zumindest Teile von Implantaten in den Scandaten enthalten sind. Beispielsweise kann bei der Angabe des Präparationstyps Implantat für einen Zahntyp ein dazugehörendes Zahnmodell zur geometrischen Analyse der Scandaten verwendet werden, bei dem ein Implantat-Teilmodell enthalten ist. Eine weitere Differenzierung des Implantat-Präparationstyps ist beispielsweise dadurch möglich, dass Bezeichnungen für den genauen Implantattyp hinzugefügt werden. Dann kann ein Implantat-Teilmodell - beispielsweise in Form eines CAD-Datensatzes - des physisch vorliegenden Implantats mit in die geometrische Analyse eingehen.

Zur geometrischen Analyse von Scandaten oraler Strukturen wird erfindungsgemäß entsprechend der zu analysierenden Scandaten eine Anzahl parametrisierter Zahnmodelle gewünschter Zahntypen ausgewählt, wobei die Parametrisierung anhand von Modellparametern erfolgt, welche Lage- und/oder Formparameter und Linienparameter umfassen und wobei jedes Zahnmodell zumindest eine parametrisierte Grenzlinie enthält, deren Verlauf durch die Linienparameter beschrieben wird und welche ein Zahnmodell in zumindest einen aktiven und zumindest einen inaktiven Anpassungsbereich unterteilt.

Die zur geometrischen Analyse vorhandenen Zahnmodelle können in einer Modelldatenbank hinterlegt sein. Dann kann eine Auswahl von Zahnmodellen derart stattfinden, dass die vorhandenen Zahntypen einer Modelldatenbank zur Auswahl einer Auswahleinheit zugeführt werden und mit Hilfe eines Auswahlsignals, beispielsweise durch die Verwendung einer grafischen Benutzeroberfläche, die gewünschten Zahntypen selektiert werden. Diese Selektion kann durch die Verwendung eines grafischen Zahnschemas übersichtlich gestaltet werden. Der Umfang der verwendeten Zahnmodelle sollte vorzugsweise dem Umfang der gescannten Zähne entsprechen, damit möglichst viel Zahnsubstanz durch das Verfahren analysiert wird. D.h. es werden in der Regel mehrere Zahnmodelle verschiedener Zahntypen zur geometrischen Analyse verwendet. Als Sonderfall kann aber auch nur ein einziges Zahnmodell ausgewählt werden.

Dabei beschreiben vorzugsweise sechs Lageparameter eines Zahnmodells die räumliche Position und Orientierung eines Zahnmodells in den Scandaten. Anatomisch sinnvolle Formvariationen der Zahnmodelle werden durch parametrisierte geometrische Transformationen erzeugt, die sich von Zahnmodell zu Zahnmodell unterscheiden können und in der Modelldatenbank hinterlegt sind. D.h., die Anzahl der Formparameter kann von Zahnmodell zu Zahnmodell variieren. Darüber hinaus können die Modellparameter neben den Lage- und/oder Formparametern und Linienparametern weitere Parameter umfassen, die z.B. Oberflächenregionen und/oder Materialeigenschaften parametrisieren.

Erfindungsgemäß werden die Zahnmodelle mit ihren Grenzlinien zur Individualisierung an die Scandaten angepasst, wobei die Individualisierung durch Variation von Modellparametern durchgeführt wird und wobei die aktiven Anpassungsbereiche der Zahnmodelle stärker gewichtet werden als die inaktiven Anpassungsbereiche. Unter Individualisierung wird die Anpassung von Zahnmodellen an Scandaten verstanden, wobei Individualisierungskriterien, wie z.B. die Anpassung von Zahnmodellen an geeignete Zielstrukturen in den Scandaten und/oder die geometrische Kopplung von Zahnmodellen untereinander, möglichst gut eingehalten werden sollen. Die erfindungsgemäße Unterscheidung von aktiven und inaktiven Anpassungsbereichen spielt bei der Anpassung der Zahnmodelle an die Scandaten eine zentrale Rolle. So umfassen optische Scandaten normalerweise keine Anteile unterhalb des Zahnfleisches und auch der Interdentalraum ist aufgrund des optischen Messprinzips oft nur teilweise enthalten. Durch die erfindungsgemäße Definition von aktiven Anpassungsbereichen wird sichergestellt, dass der gesamte aktive Modellbereich vollständig an die Scandaten angepasst werden kann. Modellanteile, die keine Entsprechungen in den Scandaten haben, werden durch die Inaktivierung vom Anpassungsprozess ausgeschlossen bzw. geringer gewichtet. Dadurch wird eine erhebliche Stabilisierung der Modellindividualisierung erreicht.

Abschließend werden auf Basis der individualisierten Zahnmodelle mit ihren individualisierten Grenzlinien die Scandaten segmentiert und/oder zumindest eine Grenzlinie in den Scandaten bestimmt. Die entsprechenden Verfahren sind dem Fachmann bekannt. Bei der Segmentierung können prinzipiell alle die Scanelemente (z.B. Vertices bei optischen Scandaten, Voxel bei radiologischen Scandaten) innerhalb der Scandaten selektiert und ihnen optional eine Segmentierungsnummer zugewiesen werden, die innerhalb der Kontur eines dazugehörenden Zahnmodells oder eines dazugehörenden Teil-Zahnmodells liegen, oder maximal um einen bestimmten Differenzwert davon abweichen. Die Selektion kann dabei in der Form vorgenommen werden, dass die betreffenden Scanelemente entfernt werden oder dass alle übrigen Scanelemente entfernt, d.h. die betreffenden Scanelemente ausgeschnitten werden. Unter "Teil-Zahnmodell" ist hierbei ein Teil eines individualisierten Zahnmodells zu verstehen, beispielsweise die Okklusionsfläche. Eventuell vorhandene Kavitäten in den Scandaten sind dadurch gekennzeichnet, dass die entsprechenden Scanelemente signifikant innerhalb eines individualisierten Zahnmodells liegen und durch eine individualisierte Präparationslinie begrenzt werden.

Die Bestimmung von Grenzlinien in den Scandaten kann durch eine Übertragung der individualisierten Grenzlinien von den Koordinatensystemen der Zahnmodelle in das Koordinatensystem der Scandaten erfolgen. Optional kann auch noch eine Feinanpassung der übertragenen Grenzlinien an die Scandaten stattfinden. So können beispielsweise die übertragenen Segmentierungslinien bei optischen Scandaten noch geringe Abstände zu den gemessenen Oberflächen in den Scandaten aufweisen. Durch eine Feinanpassung, bei der beispielsweise die Stützpunkte der Segmentierungslinien an die gemessenen Oberflächen angepasst werden, kann eine präzise Bestimmung der Segmentierungslinien in den Scandaten erfolgen.

Das erfindungsgemäße Verfahren erlaubt es die Zahnmodelle mit praktikablem Rechenaufwand und möglichst automatisiert einzusetzen. Insbesondere kann das Verfahren aufgrund des besonderen Aufbaus der Zahnmodelle zur automatischen Segmentierung und/oder Bestimmung von Grenzlinien eingesetzt werden. Dies wird durch die Stabilisierung der Individualisierung durch die gleichzeitige Anpassung von Zahnmodellen auf globaler Ebene und die Anpassung von Grenzlinien auf lokaler Ebene erreicht.

Die abhängigen Ansprüche sowie die nachfolgende Beschreibung enthalten jeweils besonders vorteilhafte Ausgestaltungen und Weiterbildungen der Erfindung, wobei insbesondere das erfindungsgemäße Analysesystem auch analog zu den Merkmalen der abhängigen Verfahrensansprüche weitergebildet sein kann. Zudem können im Rahmen der Erfindung auch die verschiedenen Merkmale unterschiedlicher Ausführungsbeispiele zu neuen Ausführungsbeispielen kombiniert werden.

Vorzugsweise erfolgt die Individualisierung von Zahnmodellen durch die Definition und Lösung einer Optimierungsaufgabe. Zur Lösung der Optimierungsaufgabe werden Modellparameter solange variiert, bis der Optimierungswert minimal oder ein Abbruchkriterium erfüllt ist. Dabei kann sich der Optimierungswert der Optimierungsaufgabe besonders bevorzugt aus einer Anzahl von Optimierungsteilwerten zusammensetzen, die jeweils bestimmten gewünschten Individualisierungskriterien entsprechen. Vorzugsweise geschieht dies durch die Berechnung einer gewichteten Summe der Optimierungsteilwerte. Die Gewichte ermöglichen dabei eine Steuerung des Einflusses der einzelnen Individualisierungskriterien. So erhält man für einen Modellparameter-Satz einen Optimierungswert.

Bevorzugte Optimierungsteilwerte beschreiben die Anpassung der Zahnmodelle an die Zähne und/oder an die Restzahnsubstanzen, d.h. die modellierten Zahnoberflächen der Zahnmodelle sollen mit den dazugehörenden unpräparierten Zahnoberflächen der Scandaten möglichst gut zur Deckung gebracht werden. Dazu kann man eine Zielstruktur in den Scandaten ermitteln, deren Berechnungsart vor allem von der aufnehmenden Modalität abhängt. Besonders einfach ist die Situation bei optischen Scandaten, wo die gemessene Oberfläche direkt als Zielstruktur verwendet werden kann. Relativ einfach ist die Zielstrukturermittlung bei CT/DVT-Scandaten, die durch Röntgenstrahlen erzeugt werden. Dort bietet sich die Verwendung eines Schwellenwertverfahrens an, bei dem die Intensitätswerte (gemessen in Hounsfield-Einheiten) der einzelnen Scanelemente daraufhin analysiert werden, ob sie einen bestimmten Schwellenwert überschreiten. Radiologisch dichte Strukturen, wie Zahnsubstanz und Knochen, lassen sich so einfach selektieren und als Zielstruktur verwenden. Etwas aufwändiger sind die Berechnungen bei MR-Scandaten, wo vorzugsweise Konturanalyseverfahren Anwendung finden, die auf Basis von Gradienten benachbarter Scanelemente arbeiten. Als Resultat erhält man für alle Modalitäten eine Oberflächen-Zielstruktur in den Scandaten, die als Zielstruktur für die Zahnmodelle verwendet werden kann.

Damit diese Zielstruktur für die Individualisierung von Segmentierungs- und Präparationslinien besonders geeignet ist, werden vorzugsweise Informationen über lokale Flächenkrümmungen genutzt. Entsprechende Berechnungsverfahren sind dem Fachmann bekannt. Diese Kenntnis von Flächenkrümmungen hat für die Individualisierung von Grenzlinien grundlegende Vorteile. Schließlich zeichnen sich die Segmentierungs- und Präparationslinien der gescannten Zähne dadurch aus, dass sie Scanelemente mit möglichst starken konkaven bzw. konvexen Flächenkrümmungen zu einer Linie verbinden.

Weitere bevorzugte Optimierungsteilwerte beschreiben die Anpassung der Zahnmodelle an die Gegenbezahnung und/oder an statische Bissregistrate und/oder an funktionelle Bissregistrate. Bei der Berechnung dieser Optimierungsteilwerte kann optional auch die Kieferbewegung mit einbezogen werden. Ein weiterer Optimierungsteilwert beschreibt die Anpassung der Zahnmodelle an nichtnatürliche orale Strukturen, beispielsweise an Implantate oder Brackets. Die dazugehörenden Zielstrukturen können insbesondere bei radiologischen CT/DVT-Scandaten in der Regel durch Schwellenwertverfahren konstruiert werden. Ein weiteres Individualisierungskriterium betrifft die mechanische Stabilität der Zahnrestaurationen, die sich aus den Zahnmodellen und Scandaten ergeben können. Der dazugehörende Optimierungsteilwert beschreibt vorzugsweise die mechanische Stabilität der Zahnrestaurationen und kann deshalb auch zur Beurteilung der Qualitäten von durchgeführten Zahnpräparationen herangezogen werden. Neben diesen beispielhaft aufgeführten funktionalen Individualisierungskriterien können aber auch ästhetische Individualisierungskriterien berücksichtigt werden, welche vor allem für Zahnrestaurationen im Frontzahnbereich wichtig sind. So kann der Patient eine besondere Form (rechteckig, dreieckig, quadratisch, schaufelförmig, etc.) der oberen Schneidezähne bevorzugen. Der dazugehörende Optimierungsteilwert kann die Abweichungen der Zahnmodelle zu den bevorzugten Formen beschreiben.

Wie oben erläutert werden in der Regel mehrere Zahntypen für die geometrische Analyse von Scandaten ausgewählt, wobei dann Gruppen von Zahnmodellen individualisiert werden können. Zur Stabilisierung der Individualisierung ist es vorteilhaft Zahnmodelle innerhalb einer Gruppe zu koppeln. Zu jeder so gebildeten Kopplungsgruppe gehört vorzugsweise ein weiterer Optimierungsteilwert. Mögliche Kopplungsgruppen sind z. B. Kontakt-, Lagen- und Formen-Kopplungsgruppen, deren dazugehörenden Kontakt-, Lagen- und Formen-Optimierungsteilwerte die Kontakt-, Lagen- und Formenbeziehungen der beteiligten Zahnmodelle beschreiben. Solche Kopplungsgruppen ermöglichen beispielsweise die Berechnung von Zahnsubstanz eines Zahntyps aus den Zahnsubstanzen anderer Zahntypen, indem beispielsweise die geometrischen Formen von Zahnmodellen gekoppelt werden. Dies ist insbesondere bei der geometrischen Analyse von Kronen- bzw. Brückenpräparationen wichtig.

Beispielsweise können bei einer Brückenpräparation die Zähne Nr. 14 und Nr. 15 fehlen, wobei die Zähne Nr. 13 und Nr. 16 unpräpariert vorhanden sind (Die Nummern sind hier die üblichen Zahnnummern des FDI-Zahnschemas). Aufgabe ist es nun, aus den die Zahnlücke begrenzenden Zähne auf die Formen der fehlenden Zähne zu schließen. Dazu kann man eine "Formen-Kopplungsgruppe" der Zahnmodelle für die Zähne Nr. 13 bis Nr. 16 bilden. Im Laufe der Individualisierung der Zahnmodelle findet eine Anpassung der Zahnmodelle für die Zähne Nr. 13 und Nr. 16 an die vorhandene Zahnsubstanz statt, was hingegen mit den Zahnmodellen für die Zähne Nr. 14 und Nr. 15 nicht möglich ist, da ja keine dazugehörende Zahnsubstanz zur Anpassung vorhanden ist. D.h. die Formen der Zahnmodelle für die Zähne Nr. 14 und Nr. 15 können sich nicht direkt durch die Anpassung an die Scandaten ergeben. Gelöst wird dieses Problem im Rahmen der Erfindung z.B. dadurch, dass sich die Formen der Zahnmodelle für die Zähne Nr. 14 und Nr. 15 indirekt aus den Formen der Zahnmodelle für die Zähne Nr. 13 und Nr. 16 ergeben. Dies kann dadurch erreicht werden, dass in die Individualisierung der Zahnmodelle ein Formen-Optimierungsteilwert eingeht, der die Kopplung der natürlichen Formen von Zahnmodellen beschreibt. Durch den Aufbau einer Modelldatenbank auf Basis von Probanden-Scandaten sind die natürlichen Formenbeziehungen von Zähnen bekannt. In dem geschilderten Beispielfall kann man zu den Zahnmodellen für die Zähne Nr. 13 und Nr. 16 denjenigen Probanden-Scandatensatz bestimmen, der am besten zu den Zahnmodellen für die Zähne Nr. 13 und Nr. 16 passt. Die dazugehörenden Probanden-Zahnmodelle für die Zähne Nr. 14 und Nr. 15 kann man dann als die gesuchten Zahnmodelle für die Brückenpräparation ansehen. Der Formen-Optimierungsteilwert beschreibt dann die Formabweichung der Zahnmodelle für die Zähne Nr. 14 und Nr. 15 zu den Probanden-Zahnmodellen für die Zähne Nr. 14 und Nr. 15.

Der Umfang einer Kopplungsgruppe kann grundsätzlich durch eine beliebige Menge unterschiedlicher Zahntypen aus der Menge der gewünschten Zahntypen festgelegt (z.B. durch die Zahntypen Nr. 13 bis Nr. 18 oder z.B. durch die Zahntypen Nr. 15 und Nr. 26) werden, d.h. die dazugehörenden Zahnmodelle müssen nicht notwendigerweise benachbart sein. Außerdem kann ein Zahnmodell auch mehreren Kopplungsgruppen gleichzeitig zugeordnet sein, z.B. einer Formen-Kopplungsgruppe und einer Kontakt-Kopplungsgruppe.

Sind Zahnmodelle benachbart, so werden die benachbarten Zahnmodelle vorzugsweise derart zu einer "Kontakt-Kopplungsgruppe" gekoppelt, dass der dazugehörende Optimierungsteilwert die Kontaktsituation der benachbarten Zahnmodelle beschreibt. Beispielsweise kann sich der Kontakt-Optimierungsteilwert aus den minimalen Entfernungen benachbarter Zahnmodelle ergeben. So ist es z.B. bei einer Brückenpräparation gewünscht, dass diese Entfernungswerte und somit der Kontakt-Optimierungsteilwert für die Zahnmodelle der Brückenkonstruktion Null sind, d.h. die Zahnmodelle der dazugehörenden Kontakt-Kopplungsgruppe sollen sich zumindest in einem Punkt berühren.

Neben Kontakt- und Formen-Kopplungsgruppen können auch "Lagen-Kopplungsgruppen" gebildet werden, derart, dass sich daraus anatomisch sinnvolle Lagebeziehungen der Zahnmodelle untereinander im Rahmen der Individualisierung ergeben. Dazu können Lagenparameter einer Lagen-Kopplungsgruppe gewünschte Lagebeziehungen der dazugehörenden Zahnmodelle definieren. Der Optimierungsteilwert für die relativen Lagen von Zahnmodellen ergibt sich vorzugsweise aus den räumlichen Relationen von anatomischen Landmarken der Zahnmodelle. Die Lagenparameter können dabei gewünschte Relationen der Landmarken untereinander beschreiben und aus den Abweichungen von diesen Relationen kann der Optimierungsteilwert für die Lagebeziehungen berechnet werden. Als Beispiel sei hier die Ausrichtung von Zahnmodell-Schneidekanten im Frontzahnbereich angeführt. Hier können alle betroffenen Frontzähne als Mitglieder einer Lagen-Kopplungsgruppe betrachtet werden. Obwohl die Formen der Zahnmodelle hier stark variieren können (rechteckig, dreieckig, etc.), ergibt die Forderung, dass die Landmarken der Zahnmodell-Schneidekanten auf einer durch die Lagenparameter parametrisierten Kurve liegen sollen, eine gute Ausrichtung der Zahnmodell-Schneidekanten im Frontzahnbereich.

Ähnlich verhält es sich mit einem Optimierungsteilwert, der die räumlichen Relationen der Formen von Zahnmodellen beschreibt. Beispielsweise kann eine Modelldatenbank aus Kieferscans von Probanden aufgebaut werden, wobei die aus einem Kieferscan erzeugten Zahnmodelle untereinander korrespondieren, da sie alle von einem Probanden stammen. Der Optimierungsteilwert für die Kopplung der Formen der Zahnmodelle beschreibt dann vorzugsweise die Abweichungen von den natürlichen Formenbeziehungen der Zahnmodelle innerhalb einer Kopplungsgruppe. Beispielsweise kann für ein Ausgangs-Zahnmodell, welches zu einer Formen-Kopplungsgruppe gehört, ein Probanden-Zahnmodell mittels der Modelldatenbank bestimmt werden, das sich morphologisch möglichst wenig vom Ausgangs-Zahnmodell der Formen-Kopplungsgruppe unterscheidet. Zu diesem Probanden-Zahnmodell können dann die korrespondierenden Probanden-Zahnmodelle, d.h. die vom gleichen Probanden stammenden Zahnmodelle, ermittelt werden. Aus den morphologischen Abweichungen der Zahnmodelle der Formen-Kopplungsgruppe zu den dazugehörenden korrespondierenden Probanden-Zahnmodelle kann dann ein so genannter "Korrespondenz-Abweichungswert" für das Ausgangs-Zahnmodell der Formen-Kopplungsgruppe berechnet werden. Dieser Vorgang wird vorzugsweise für jedes Zahnmodell der Kopplungsgruppe wiederholt und aus den Korrespondenz-Abweichungswerten kann dann eine Bestimmung des Optimierungsteilwerts für die Formenbeziehungen erfolgen.

Der Umfang zumindest einer Kopplungsgruppe sollte vorzugsweise dem Umfang der gescannten Zähne entsprechen, damit möglichst viel Zahn- und/oder Restzahnsubstanz durch das Verfahren analysiert wird. Insbesondere bei vorhandenen Zahnpräparationen wird aus der Zahn- und/oder Restzahnsubstanz aller gescannten Zähne auf die Formen der Zahnrestaurationen geschlossen. Je größer dabei der Zahndefekt für einen Zahntyp ist, desto wichtiger ist die Analyse der Zahn- und/oder Restzahnsubstanzen anderer Zahntypen. Insbesondere wenn aber nur ein minimaler Defekt vorliegt, z.B. bei einer Inlaypräparation ohne Höckerbeteiligung, so funktioniert das erfindungsgemäß vorgeschlagene Verfahren auch mit einem einzigen Zahntyp ausgezeichnet. Alternativ können aber auch bei der Verwendung von mehreren Zahntypen zur Analyse einer Situation mit geringen und/oder keinen Zahndefekten die Kopplungs-Optimierungsteilwerte sehr gering oder sogar mit Null gewichtet sein.

Zur Stabilisierung der Zahnmodellindividualisierung kann der Bediener in einer bevorzugten Variante des Verfahrens interaktiv Strukturen in den Scandaten markieren, zu denen Optimierungsteilwerte berechnet werden, die die Abweichungen der markierten Strukturen zu korrespondierenden Strukturen der Zahnmodelle beschreiben. Wichtige markierbare Strukturen in den Scandaten sind unpräparierte Oberflächen von Zähnen, Restzahnsubstanzen, Segmentierungs-, bzw. Präparationslinien von gescannten Zähnen, anatomische Landmarken, nichtnatürliche orale Strukturen, Kontaktpunkte zu Nachbarzähnen und Kontaktpunkte zur Gegenbezahnung. Die Individualisierung wird dann dadurch stabilisiert, dass die Entfernungen der markierten Strukturen zu den dazugehörenden Strukturen der Zahnmodelle während der Optimierung minimiert werden. Dieses Vorgehen ist insbesondere dann sinnvoll, wenn starke Normabweichungen bei den gescannten Zähnen, oder deren relativen Lagen zueinander, vorhanden sind. Es können aber auch Messartefakte in den Scandaten vorliegen oder der Verlauf der Präparationslinien ist durch mangelhaft ausgeführte Präparationen nicht eindeutig definiert.

Die interaktive Markierung von Strukturen in den Scandaten zur Unterstützung des Individualisierungsvorgangs erfolgt vorzugsweise mittels Mauseingaben einer grafischen Benutzeroberfläche. Die Art der zu markierenden Struktur ist stark abhängig vom Individualisierungskriterium, wobei die Berechnungsart des Optimierungsteilwertes bei allen Individualisierungskriterien mit Bediener-Interaktion sehr ähnlich ist. Zur Berechnung der Optimierungsteilwerte werden in einer bevorzugten Variante die minimalen Entfernungen der markierten Strukturen in den Scandaten zu den dazugehörenden Strukturen der Zahnmodelle berechnet. Ein Optimierungsteilwert ergibt sich dann über die Summe der Quadrate solcher Entfernungswerte.

Bei einer bevorzugten Variante des Verfahrens wird nach Bestimmung der individualisierten Zahnmodelle und vor der weiteren geometrischen Analyse der Scandaten eine Feinanpassung der individualisierten Zahnmodelle mit ihren individualisierten Grenzlinien untereinander und/oder an die Scandaten durchgeführt. Dies ist vorteilhaft, da die Formvielfalt der Zahnmodelle mit ihren Grenzlinien durch die Anzahl der Formparameter bzw. Linienparameter praktisch beschränkt ist und insbesondere die Präparationslinien mit hoher Präzision berechnet werden sollten. Von Vorteil ist die Anwendung von Deformationstransformationen, welche eine Feinanpassung mit möglichst geringen Verschiebungswerten vornehmen und dabei keine Kanten oder Faltungen erzeugen. Diese Anpassungen können analog zur Individualisierung der Zahnmodelle durchgeführt werden, wobei als Optimierungsparameter jetzt die Parameter dieser Deformationstransformationen Verwendung finden. Die Berechnung des zu minimierenden Optimierungswertes aus Optimierungsteilwerten kann unverändert übernommen werden.

Die geometrisch deformierbaren Zahnmodelle in einer Modelldatenbank können nach verschiedenen Prinzipien aufgebaut sein. Da es sich bei den Zielstrukturen in den Scandaten vor allem um Grenzflächen handelt, bieten sich Oberflächenmodelle als Zahnmodelle an. Die geometrische Modellierung der Modelloberflächen kann durch einfache Triangulierung erfolgen, oder aber auch durch aufwändigere Modellierungen höherer Ordnung (Bezier-, Nurbs-, B-Spline-Modelle, etc.). Mit mehr Rechen- und Speicheraufwand als bei den Oberflächenmodellen ist hingegen die Verwendung von Volumenmodellen (Voxel-, FEM-Modellen, etc.) verbunden. Mit ihnen lassen sich gut innere Strukturen von Zähnen aber auch mechanische Eigenschaften modellieren. Von Vorteil ist die Markierung von anatomischen Landmarken, Zahnachsen, Richtungsbezeichnungen, Oberflächenregionen und/oder weiteren charakteristischen geometrischen Strukturen an den Zahnmodellen, da man diese Markierungen nach erfolgreicher Individualisierung der Zahnmodelle auf die Scandaten übertragen kann. Auf Basis dieser individualisierten geometrischen Strukturen kann auch eine Vermessung der Scandaten vorgenommen werden.

Die Zahnmodelle einer Modelldatenbank können in verschiedenen Auflösungsstufen vorliegen. Es ist dann von Vorteil, das erfindungsgemäße Verfahren zuerst mit den Zahnmodellen der gröbsten Auflösungsstufe durchzuführen, wobei die Auflösungsstufe in späteren Schritten, nach erfolgreichem Durchlauf, erhöht wird. Dies ermöglicht vor allem eine Geschwindigkeitssteigerung des Verfahrens. Dabei kann die Datenbank z.B. auch komplette Sätze von Zahnmodellen in unterschiedlichen Auflösungsstufen umfassen, bzw. Teildatenbanken mit jeweils unterschiedlichen Auflösungsstufen. Nach einer kompletten Optimierung, d.h. nach der Individualisierung von Zahnmodellen aus einer Daten- oder Teildatenbank mit einer niedrigen Auflösungsstufe wird dann das Verfahren noch einmal mit einer Daten- oder Teildatenbank mit einer höheren Auflösungsstufe durchgeführt. Dabei können die individualisierten Zahnmodelle aus einem vorhergehenden Durchlauf als Startmodelle für den nachfolgenden Durchlauf verwendet werden. Ebenso kann bei der Lösung der Optimierungsaufgabe mit verschiedenen Auflösungsstufen gearbeitet werden.

Die geometrischen Transformationen von Zahnmodellen werden durch Formparameter beschrieben, wobei diese Parametrisierung auf unterschiedliche Weise realisiert werden kann. Vorzugsweise finden Transformationen Verwendung, bei denen die Formparameter der Zahnmodelle entsprechend ihres Einflusses auf die Zahnmodellgeometrie geordnet sind. D.h. die wichtigen Formparameter stehen am Anfang der Formparameterliste und werden bei der Zahnmodellindividualisierung zuerst optimiert, bevor Formparameter optimiert werden, die Details der Zahnmodelle parametrisieren. Dadurch wird eine Geschwindigkeitssteigerung und Stabilisierung der Zahnmodellindividualisierung erreicht. Der Definitionsbereich der Formparameter ist dabei vorzugsweise nicht beliebig, sondern wird bevorzugt durch die Analyse von Trainingsdaten bestimmt. Auf diese Weise wird erreicht, dass nur anatomisch sinnvolle Zahnmodelle durch die Formparameter erzeugt werden. Von besonderem Vorteil für das erfindungsgemäß vorgeschlagene Verfahren ist die Verwendung von parametrisierten dreidimensionalen Transformationsfeldern für die Zahnmodelle. Ein dreidimensionales Transformationsfeld besteht aus Verschiebungsvektoren für die Vertices (Stützpunkte) eines Zahnmodells. Diese Verschiebungsvektoren können auch parametrisiert werden, wie z.B. durch die parametrisierte Verschiebung von anatomischen Landmarken eines Zahnmodells. Die Verschiebungsvektoren aller Vertices ergeben sich dann beispielsweise aus den Entfernungen der Vertices zu den verschobenen anatomischen Landmarken. Hingegen ist bei Anwendungen des vorgeschlagenen Verfahrens, bei denen weniger die Ansprüche an die Genauigkeit, sondern eher schnell zu erzielende Ergebnisse im Vordergrund stehen, die Verwendung von Formparametern vorteilhaft, die geometrischen Konstruktionsparametern entsprechen (z.B. Zahnbreite, -tiefe, Höckerabstände, Schneidekantendicke, Wurzellängen, etc.).

Eine weitere bevorzugte Variante des erfindungsgemäßen Verfahrens ist dadurch gekennzeichnet, dass die Grenzlinien auf den Oberflächen der Zahnmodelle liegen und/oder dass die Grenzlinien einen parametrisierten Abstand zu den Oberflächen der Zahnmodelle aufweisen und/oder dass die Linienparameter so gewählt werden, dass die Grenzlinien sich nicht schneiden und/oder einen Mindestabstand zueinander einhalten. Die Möglichkeit, dass die Grenzlinien einen parametrisierten Abstand zu den Oberflächen der Zahnmodelle aufweisen, erhöht die Verlaufsvielfalt der Grenzlinien. Dieser Ansatz ist insbesondere dann sinnvoll, wenn Zahnmodelle aus einer Modelldatenbank Verwendung finden, die aus der Analyse einer relativ kleinen Menge von Probanden-Scandaten entstanden ist. Zu beachten ist, dass die Vertices einer Grenzlinie in der Regel unterschiedliche Abstände zu dem dazugehörenden Zahnmodell aufweisen können. Von Vorteil ist die Vorgabe eines maximalen Abstandwertes um die Abstände zu beschränken und/oder die Berechnung eines Optimierungsteilwertes, der sich aus den Abstandswerten ergibt. Eine sinnvolle Beschränkung der Linienparameter wird dadurch erreicht, dass im Optimierungsverfahren die Randbedingung vorgegeben wird, dass sich die Grenzlinien nicht schneiden und/oder einen Mindestabstand zueinander einhalten sollen. Bei einem präparierten Zahn liegt in der Regel die Präparationslinie oberhalb der Segmentierungslinie, bzw. kann in Sonderfällen diese höchstens berühren. Die Berücksichtigung dieser Randbedingungen führt zu einer Stabilisierung der Zahnmodellindividualisierung.

Bei einer besonders bevorzugten Verfahrensvariante umfassen die Grenzlinien eines Zahnmodells zumindest eine Segmentierungslinie und/oder zumindest eine Präparationslinie. Bei optischen Scandaten beschreibt die wichtigste Segmentierungslinie den Übergang von Zahnsubstanz zu Zahnfleisch, der sich in der Regel durch eine konkave Oberflächenkrümmung auszeichnet. Bei radiologischen Scandaten kann man neben dieser Segmentierungslinie auch eine Segmentierungslinie verwenden, die den Übergang von Zahnsubstanz zu Kieferknochen beschreibt, wobei sich dieser Übergang ebenfalls durch konkave Oberflächenkrümmungen auszeichnet. Weitere Segmentierungslinien können den Kontakt benachbarter Zähne beschreiben. Sofern kein Kontakt vorhanden ist, kann solch eine Segmentierungslinie in einen Punkt übergehen, so dass der dazugehörende umschlossene inaktive Anpassungsbereich den Flächeninhalt Null besitzt. Bei kieferorthopädischen Fragestellungen können in den Scandaten beispielsweise auch Brackets enthalten sein. Es ist dann von Vorteil, die Kontaktfläche der Brackets zu den Zähnen durch entsprechende Segmentierungslinien zu beschreiben. Auch hier ist der Übergang von Zahnsubstanz zu den Brackets durch konkave Oberflächenkrümmungen charakterisiert.

Von zentraler Bedeutung kann bei der Analyse von Scandaten präparierter Zähne die Verwendung von Präparationslinien sein, welche die Kavitäten der präparierten Zähne begrenzen. Im Gegensatz zu den bisher beschriebenen Segmentierungslinien zeichnet sich der Übergang von unpräparierter Zahnsubstanz zu einer Kavität durch konvexe Oberflächenkrümmungen aus. Zur Individualisierung der Grenzlinien werden aufgrund dieser Zusammenhänge vorzugsweise Zielstrukturen in den Scandaten bestimmt, die entsprechende Oberflächenkrümmungen aufweisen. Zur Individualisierung der Segmentierungslinien wird eine Segmentierungslinien-Zielstruktur bestimmt, die aus Teilen von Scandaten besteht und zumindest eine konkave Oberflächenstruktur umfasst. Hingegen wird zur Individualisierung der Präparationslinien eine Präparationslinien-Zielstruktur bestimmt, die aus Teilen von Scandaten besteht und zumindest eine konvexe Oberflächenstruktur umfasst.

Vorzugsweise liegen bei einer weiteren Variante des Verfahrens die Anpassungsbereiche auf den Zahnmodelloberflächen und die aktiven Anpassungsbereiche werden bei optischen Scandaten an unpräparierte Zahnoberflächen in den Scandaten angepasst und bei radiologischen Scandaten an unpräparierte Zahnoberflächen, die außerhalb des Zahnfleisches oder außerhalb des Kieferknochens liegen oder aus Zahnschmelz bestehen. Dabei spielt es keine Rolle, ob die verwendeten Zahnmodelle Oberflächen-, bzw. Volumenmodelle sind. Es ist vor allem wichtig, dass die aktiven Anpassungsbereiche Oberflächenbereiche der Zahnmodelle sind und unpräparierten Zahnoberflächen in den Scandaten entsprechen. D.h. die aktiven Anpassungsbereiche sollen vorzugsweise im Rahmen der Individualisierung möglichst vollständig an sämtliche vorhandene unpräparierte Zahnoberflächen in den Scandaten angepasst werden. Bei erfolgreicher Individualisierung der Zahnmodelle entsprechen dann die individualisierten aktiven Anpassungsbereichen den unpräparierten Zahnoberflächen. Die Ausdehnungen der aktiven Modellbereiche wird hierbei über parametrisierte Grenzlinien definiert, wobei die Grenzlinien so zu wählen sind, dass sie bei der Individualisierung an Zielstrukturen in den Scandaten angepasst werden, welche unpräparierte Zahnoberflächen begrenzen. Diese Zielstrukturen umfassen bei optischen Scandaten beispielsweise den konkaven Übergang von Zahnfleisch zu unpräparierter Zahnoberfläche und den konvexen Übergang von Kavität zu unpräparierter Zahnoberfläche. Bei radiologischen Scandaten kann als konkaver Übergang auch der Übergang von Kieferknochen zu unpräparierter Zahnsubstanz verwendet werden, aber auch der Übergang von Zahnschmelz zu Dentin im Bereich der Zahnhälse. Die Unterscheidung von Zahnschmelz und Dentin kann dabei z. B. über die Hounsfield-Werte der Scanelemente erfolgen.

Eine weitere bevorzugte Variante des erfindungsgemäßen Verfahrens ist dadurch gekennzeichnet, dass für die Individualisierung von Zahnmodellen ein Qualitätswert berechnet wird, der sich aus einzelnen Qualitätsteilwerten ergibt, die die Erfüllung von Individualisierungskriterien beschreiben. Der Qualitätswert einer Individualisierung beschreibt dann quantitativ, wie gut die berücksichtigten Individualisierungskriterien eingehalten wurden. Ein Qualitätswert von 100% sollte für die Individualisierung von Zahnmodellen verwendet werden, bei der alle Individualisierungskriterien vollständig erfüllt sind. Geringere Prozentwerte beschreiben eine Individualisierung, bei der einzelne Individualisierungskriterien nur teilweise oder überhaupt nicht erfüllt werden. Beispielsweise kann ein individualisiertes Zahnmodell am Verfahrensende immer noch eine Abweichung der Zahnmodelloberfläche zu den Scandaten aufweisen. Aus der mittleren Abweichung der Vertices der Zahnmodelloberfläche kann dann ein Qualitätsteilwert durch eine nichtlineare Umrechnung berechnet werden. So kann man z.B. eine Umrechnungstabelle verwenden, bei der mittlere Abweichungswerte Qualitätsteilwerten zugeordnet werden. Ein mittlerer Abweichungswert sollte dann einem Qualitätsteilwert von 100% entsprechen und mittlere Abweichungswerte über einem relativ großen Schwellenwert (z.B. 1cm) sollten Qualitätswerten von 0% entsprechen. Die Berechnung eines Qualitätswertes aus Qualitätsteilwerten geschieht vorzugsweise durch die Berechnung einer gewichteten Summe der Qualitätsteilwerte. Die Gewichtungsfaktoren ermöglichen dabei eine Steuerung des Einflusses der einzelnen Individualisierungskriterien. Vorzugsweise werden die Gewichtungsfaktoren so gewählt, dass alle Individualisierungskriterien gleichermaßen gut berücksichtigt werden.

Sofern die Individualisierung der Zahnmodelle durch Lösung einer Optimierungsaufgabe durchgeführt wird, kann man vorzugsweise die Qualitätsteilwerte aus den Optimierungsteilwerten bestimmen, wobei beide Werte zu dem gleichen Individualisierungskriterium gehören. Dabei kann ein Qualitätsteilwert aus dem dazugehörenden Optimierungsteilwert durch eine im Allgemeinen nichtlineare Umrechung bestimmt werden. Einem minimal möglichen Optimierungsteilwert entspricht in diesem Fall einem Qualitätsteilwert von 100% und einem maximal möglichen Optimierungsteilwert einem Qualitätsteilwert von 0%.

Prinzipiell kann aber auch ein Optimierungsteilwert direkt als ein Qualitätsteilwert gesehen werden. Ein Vorteil in der Verwendung von umgerechneten Qualitätsteilwerten gegenüber der Verwendung der unveränderten Optimierungsteilwerte selber ist die bessere Anschaulichkeit für den Bediener. So ist es besser verständlich von einer 85% Anpassung eines Zahnmodells an die Zahn-, bzw. Restzahnsubstanz zu sprechen, als von einem mittleren Entfernungswert der Zahnmodellvertices zur Zahn-, bzw. Restzahnsubstanz von beispielsweise 158µm. Im Folgenden wird daher der Begriff Qualitätsteilwert verwendet auch wenn, soweit nicht explizit anders erwähnt, ein Optimierungsteilwert hiermit umfasst sein kann.

Die Qualitätsteilwerte (bzw. entsprechend die Optimierungsteilwerte) entscheiden vorzugsweise darüber, ob der Bediener zur Markierung von Strukturen in den Scandaten aufgefordert wird, die bei einem nochmaligen Ablauf des Verfahrens mit in die Individualisierung von Zahnmodellen eingehen. Liegt beispielsweise der Qualitätsteilwert für die Anpassung eines Zahnmodells an die Zahn und/oder Restzahnsubstanz unterhalb (bzw, oberhalb bei der Verwendung von Optimierungsteilwerten als Qualitätsteilwerte) einer vorgegebenen Schwelle, so wird der Bediener zur Markierung des entsprechenden Zahns und/oder der Restzahnsubstanz aufgefordert. Durch diese Markierung ist ein neues Individualisierungskriterium entstanden, nämlich die Anpassung von einem zu der Markierung gehörenden Zahnmodells an diese Markierung. Danach wird das erfindungsgemäße Verfahren erneut gestartet und überprüft, ob alle Individualisierungskriterien - einschließlich des neuen Individualisierungskriteriums - mit ausreichender Qualität erfüllt sind und ob evtl. weitere Bediener-Eingaben erforderlich sind. Dieses Vorgehen wird vorzugsweise iterativ wiederholt bis ein Abbruchkriterium erfüllt ist. Ein solches Abbruchkriterium kann z.B. sein, dass alle Qualitätsteilwerte einen Schwellenwert überschritten (bzw. unterschritten) haben oder ob eine vorgegebene Anzahl von Iterationsschritten erreicht wurde.

Die Gewichtung von Anpassungsbereichen der Zahnmodelle kann bei der Individualisierung konstant sein, aber auch während des Individualisierungsvorgangs verändert werden. Eine Verfahrensvariante ist dadurch gekennzeichnet, dass für ein Zahnmodell die Gewichtung der Anpassungsbereiche auf Basis der Qualitätsteilwerte für die Individualisierung der Grenzlinien durchgeführt wird, wobei mit steigenden Qualitätsteilwerten für die Individualisierung der Grenzlinien die aktiven Anpassungsbereiche bei der Anpassung an die Scandaten stärker gewichtet werden. Dieses Vorgehen ist insbesondere dann von Vorteil, wenn der Individualisierungsvorgang mit in den Scandaten positionierten Zahnmodellen startet, die noch starke Abweichungen zu dazugehörenden Zielstrukturen in den Scandaten aufweisen. In der Regel sind dann auch die Grenzlinien noch nicht gut an die Scandaten angepasst. D.h. die aktiven Anpassungsbereiche der Zahnmodelle können sich in ihrer Ausdehnung noch relativ stark von den unpräparierten Zahnoberflächen in den Scandaten unterscheiden. Erst wenn die Grenzlinien relativ gut an die Scandaten angepasst sind, was sich durch entsprechend hohe Qualitätsteilwerte für die Individualisierung der Grenzlinien ausdrückt, kann man davon ausgehen, dass auch die aktiven Anpassungsbereiche relativ gut den unpräparierten Zahnoberflächen entsprechen. Dann ist es sinnvoll, diese Anpassungsbereiche bei der Individualisierung stärker zu gewichten.

Eine weitere Verfahrensvariante ist dadurch gekennzeichnet, dass anatomische Landmarken und/oder Zahnachsen und/oder Richtungsbezeichnungen und/oder Oberflächenregionen und/oder weitere charakteristische geometrische Strukturen an den Zahnmodellen markiert und nach der Individualisierung auf die Scandaten übertragen werden und/oder das auf Basis dieser individualisierten Strukturen eine geometrische Vermessung der Scandaten vorgenommen wird. Dabei kann der Markierungsvorgang interaktiv und/oder algorithmisch erfolgen. Bei einem interaktiven Vorgehen ist es von Vorteil eine grafische Benutzeroberfläche zur Erstellung der Markierungen zu benutzen. Zeitsparender und vorzuziehen ist der Einsatz von geeigneten Algorithmen um an Zahnmodellen charakteristische geometrische Strukturen zu markieren. Entsprechende Verfahren sind dem Fachmann bekannt. Von besonderem Vorteil ist ein Vorgehen, bei dem die Markierungen nach der Individualisierung der Zahnmodelle und vor der Übertragung auf die Scandaten algorithmisch bestimmt werden. Dieses Vorgehen ermöglicht eine präzise Bestimmung von charakteristischen geometrischen Strukturen in den Scandaten, auch wenn die geometrisch deformierbaren Zahnmodelle während des Individualisierungsvorgangs ihre charakteristische Morphologie verändern.

Bei einer besonders bevorzugten Variante des erfindungsgemäßen Verfahrens werden aus den individualisierten Zahnmodellen und Scandaten virtuelle Zahnrestaurationen von präparierten Zähnen bestimmt. Aus den charakteristischen geometrischen Formen der ermittelten virtuellen Zahnrestaurationen und/oder der zu den individualisierten Zahnmodellen dazugehörenden individualisierten Grenzlinien können dann bevorzugt die Präparationstypen der zu den Zahnmodellen korrespondierenden Zähne bestimmt werden. Optional können sprachliche und/oder symbolische Bezeichnungen für diese ermittelten Präparationstypen mit einem Zahnschema und/oder mit einem reduzierten Zahnschema und/oder grafisch mit den Scandaten dargestellt werden.

Geeignete Verfahren zur Bestimmung von virtuellen Zahnrestaurationen aus individualisierten Zahnmodellen und Scandaten sind dem Fachmann bekannt. Dazu werden prinzipiell die Präparationslinien der Scandaten in geeigneter Weise möglichst stetig und glatt mit den dazugehörenden Zahnmodellen verbunden und dann die Kavitäten der präparierten Zähne als untere Begrenzungen hinzugefügt. Als Ergebnis erhält man für die virtuellen Zahnrestaurationen dreidimensionale Modelle von Schleifkörpern, die maschinell hergestellt werden können. Man kann aber auch die virtuellen Zahnrestaurationen mehrteilig gestalten, indem zuerst ein Trägergerüst und danach eine Oberkonstruktion mit den Kauflächen bestimmt wird.

Aus den individualisierten Zahnmodellen mit ihren individualisierten Grenzlinien und/oder den dazugehörenden virtuellen Zahnrestaurationen lassen sich aber auch die Präparationstypen der gescannten Zähne bestimmen. Der einfachste Fall liegt vor, wenn keine Präparationslinie für einen Zahntyp gefunden wurde, dann ist der entsprechende Zahn auch nicht präpariert und es ist auch keine Kavität vorhanden. Im Rahmen dieser Erfindung wird aufgrund dieser Zusammenhänge ergänzend zu den Präparationstypen Inlay-, Krone-, etc. der Präparationstyp "Zahn" für eine fehlende Präparation verwendet. Bei vorhandener Präparation werden hingegen aus den charakteristischen geometrischen Formen der ermittelten virtuellen Zahnrestaurationen und/oder der dazugehörenden individualisierten Grenzlinien die Präparationstypen (Inlay, Krone, Brücke,...) der gescannten präparierten Zähne bestimmt.

Bei einem erfindungsgemäßen Verfahren zur Erzeugung einer Modelldatenbank, die zur Verwendung in dem oben beschrieben Verfahren für verschiedene Zahntypen jeweils eine Anzahl von parametrisierten Zahnmodellen (d. h. mindestens ein Zahnmodell) enthält, erfolgt die Parametrisierung anhand von Modellparametern, welche Lage- und/oder Formparameter und Linienparameter umfassen und wobei jedes Zahnmodell zumindest eine parametrisierte Grenzlinie enthält, deren Verlauf durch die Linienparameter beschrieben wird und welche ein Zahnmodell in zumindest einen aktiven und zumindest einen inaktiven Anpassungsbereich unterteilt.

Bei einer bevorzugten Variante des Verfahrens zur Erzeugung einer Modelldatenbank werden dabei die ganze Modelldatenbank oder zumindest Teile der Modelldatenbank durch die Analyse einer Menge von optischen und/oder radiologischen Scandaten künstlicher und/oder natürlicher oraler Strukturen aufgebaut. Künstliche orale Strukturen umfassen im Sinne der Erfindung künstliche Zähne, Zahngruppen, Gebisse aber auch Implantate, Abutments und weitere Verankerungssysteme. In der Regel liegen solche künstlichen oralen Strukturen in einer Vielzahl vorkonfektionierter Ausprägungen von verschiedenen kommerziellen Anbietern vor. Hingegen umfassen natürliche orale Strukturen im Sinne der Erfindung natürliche Zähne, Zahngruppen, Gebisse aber auch Zahnfleisch, Kieferknochen, Nerven und weitere anatomische Strukturen. Vorzugsweise erhält man die optischen Scandaten natürlicher oraler Strukturen aus optischen Scans von Gipsabdrücken karies-, bzw. defektfreier Ober- und Unterkiefer. Es können aber auch radiologische Scandaten von Kiefern verwendet werden, wobei die Scandaten eine geringere Genauigkeit als die optischen Scandaten aufweisen, dafür aber subgingivale und intradentale Strukturen enthalten. Eine auf der Basis von CT/DVT-Scans aufgebaute Modelldatenbank ermöglicht die Modellierung von Zahnmodellen, die Wurzelstrukturen enthalten. Solche Zahnmodelle sind dann vorzugsweise zur geometrischen Analyse von radiologischen Scandaten geeignet.

In der Regel müssen die Scandaten, insbesondere wenn es sich um Scandaten natürlicher oraler Strukturen handelt, in einem ersten Schritt des Datenbankaufbaus zuerst segmentiert werden, um Scandaten eines gewünschten Zahntyps zu erhalten. Dieser Schritt entfällt meist bei Scandaten künstlicher oraler Strukturen, da diese in der Regel physisch separiert vorliegen und einzeln eingescannt werden können. In beiden Fällen erhält man aus der Vermessung physisch vorhandener oraler Strukturen eine Menge von Scandaten eines gewünschten Zahntyps, die zum Aufbau einer Modelldatenbank verwendet werden kann.

Sofern sowohl optische als auch radiologische Scandaten der einzelnen Probanden vorliegen, ist es vorteilhaft, beide Scandatenarten beim Aufbau einer Modelldatenbank nach der Segmentierung zu kombinieren. Dazu bildet man vorzugsweise Paare von optischen und radiologischen Scandaten des gleichen Zahntyps eines Probanden und registriert diese. Dies kann dadurch erreicht werden, dass die beiden Scandatensätze eines Paars durch die Bestimmung optimaler Translations- und Rotationswerte möglichst gut zur Deckung gebracht werden. Als Ergebnis erhält man kombinierte optisch-radiologische Scandatensätze, die ebenso zum Aufbau einer Modelldatenbank geeignet sind. Die auf dieser Datenbasis konstruierten Zahnmodelle kombinieren die hohe Genauigkeit optisch vermessener Zahnoberflächen mit radiologisch gemessenen subgingivalen Strukturen.

In dem sich anschließenden zweiten Schritt des Datenbankaufbaus können die Zahnmodelle eines Zahntyps konstruiert werden, d.h. es findet eine Anpassung von Oberflächen- bzw. Volumen-Zahnmodellen (einer gewünschten Auflösung) an die Scandaten eines gewünschten Zahntyps statt. Falls im ersten Schritt des Datenbankaufbaus eine Segmentierung durchgeführt wurde, sind in der Regel auch die Segmentierungslinien in den Scandaten bekannt, welche dann nach der Konstruktion der Zahnmodelle auf diese übertragen werden können. Alternativ kann aber auch eine interaktive Markierung der Segmentierungslinien an den Zahnmodellen stattfinden. Optional können in diesem Schritt des Datenbankaufbaus auch anatomische Landmarken, Zahnachsen, Richtungsbezeichnungen, Oberflächenregionen und/oder weitere charakteristische geometrische Strukturen für die Zahnmodelle algorithmisch und/oder interaktiv bestimmt werden. Die dazu notwendigen Verfahren sind dem Fachmann bekannt.

Eine Analyse von morphologischen Abweichungen der Zahnmodelle eines Zahntyps untereinander kann im dritten Schritt des Datenbankaufbaus durchgeführt werden, indem für jedes mögliche Paar von Zahnmodellen ein morphologischer Abweichungswert berechnet wird. Dies kann dadurch erreicht werden, dass die beiden Zahnmodelle eines Paars durch die Bestimmung optimaler Translations- und Rotationswerte möglichst gut zur Deckung gebracht werden und dass der morphologische Abweichungswert die noch bestehende morphologische Abweichung der beiden Zahnmodelle beschreibt.

Im vierten Schritt des Datenbankaufbaus kann ein Mittelwert-Zahnmodell eines Zahntyps durch eine Analyse der berechneten morphologischen Abweichungswerte ausgewählt werden. Aufgrund der Abweichungsanalyse der Zahnmodelle untereinander erhält man für jedes der n Zahnmodelle eines Zahntyps (n-1) morphologische Abweichungswerte. Als Mittelwert-Zahnmodell wird vorzugsweise dasjenige Zahnmodell angesehen, dessen Summe der Quadrate der morphologischen Abweichungswerte minimal ist.

Es kann dann bevorzugt eine Sortierung der Zahnmodelle eines Zahntyps im fünften Schritt des Datenbankaufbaus erfolgen. Besonders bevorzugt wird die Sortierung dadurch erzeugt, dass ausgehend vom Mittelwert-Zahnmodell des Zahntyps die restlichen Zahnmodelle des Zahntyps, auf Basis der morphologischen Abweichungswerte zu den bereits sortierten Zahnmodellen, an die Sortierung angefügt werden. Vorzugsweise wird immer dasjenige Zahnmodell an die Sortierung angefügt, dessen Summe der Quadrate der morphologischen Abweichungswerte zu den bereits sortierten Zahnmodellen maximal ist. Durch dieses Vorgehen ist sichergestellt, dass am Anfang der Sortierung diejenigen Zahnmodelle stehen, die sich aufgrund ihrer Morphologie am meisten unterscheiden.

In einem abschließenden sechsten Schritt des Datenbankaufbaus wird vorzugsweise eine geometrische Transformation zu dem Mittelwert-Zahnmodell eines Zahntyps hinzugefügt, um auch Zwischenformen von Zahnmodellen für die Individualisierung zu erzeugen. Für ein Mittelwert-Zahnmodell wird eine geeignete geometrische Transformation bevorzugt durch die Forderung definiert, dass die Form des Mittelwert-Zahnmodells in zumindest ein in der Sortierung folgendes Ziel-Zahnmodell kontinuierlich überführt werden kann. Durch dieses Vorgehen ist sichergestellt, dass die dazugehörenden Formparameter effektiv variiert werden können und dass mit steigender Anzahl der Formparameter die Formvielfalt des transformierten Mittelwert-Zahnmodells erhöht wird. Die Anzahl der Ziel-Zahnmodelle kann dabei vom Verwendungszweck der Modelldatenbank abhängen. Da am Anfang der Sortierung diejenigen Zahnmodelle stehen, deren Morphologien sich am stärksten unterscheiden, kann durch die Berücksichtigung einer relativ kleinen Anzahl von Ziel-Zahnmodellen eine Reduktion des Speicherumfangs der Modelldatenbank erreicht werden. Beispielsweise können bei einer geometrischen Analyse von Scandaten, wo eher eine hohe Geschwindigkeit im Vordergrund steht, geometrische Transformationen von Mittelwert-Zahnmodellen verwendet werden, die aus einer kleineren Anzahl von Ziel-Zahnmodellen entstanden sind. Der dazugehörende Speicherbedarf für die geometrischen Transformationen in der Modelldatenbank ist dann relativ gering.

Ein erfindungsgemäßes Analysesystem zur geometrischen Analyse von Scandaten oraler Strukturen benötigt zur Umsetzung des Verfahrens eine Schnittstelle zum Empfang der von einer Modalität gemessenen Scandaten, eine Auswahleinheit zur Auswahl der vom Verfahren zu verwendenden Zahntypen, eine Speichereinrichtung mit einer Modelldatenbank, die für verschiedene Zahntypen jeweils eine Anzahl von parametrisierten Zahnmodellen enthält, wobei die Parametrisierung anhand von Modellparametern erfolgt, welche Lage- und/oder Formparameter und Linienparameter umfassen und wobei jedes Zahnmodell zumindest eine parametrisierte Grenzlinie enthält, deren Verlauf durch die Linienparameter beschrieben wird und welche ein Zahnmodell in zumindest einen aktiven und zumindest einen inaktiven Anpassungsbereich unterteilt. Außerdem benötigt das Analysesystem eine Individualisierungseinheit, bei der die Zahnmodelle mit ihren Grenzlinien zur Individualisierung an die Scandaten anpasst werden, wobei diese Individualisierung durch Variation von Modellparametern durchgeführt wird und wobei die aktiven Anpassungsbereiche der Zahnmodelle stärker gewichtet werden als die inaktiven Anpassungsbereiche. Schließlich benötigt das Analysesystem eine Analyseeinheit, bei der auf Basis der individualisierten Zahnmodelle mit ihren individualisierten Grenzlinien die Scandaten segmentiert werden und/oder zumindest eine Grenzlinie in den Scandaten bestimmt wird.

Auswahl-, Individualisierungs- und Analyseeinheit des Analysesystems können besonders bevorzugt in Form von Software auf einem entsprechend geeigneten Prozessor eines Computers realisiert werden. Dieser Computer sollte eine entsprechende Schnittstelle zum Empfang der Scandaten und eine geeignete Speichereinrichtung für eine Modelldatenbank aufweisen. Dabei muss diese Speichereinrichtung nicht notwendigerweise integrierter Bestandteil des Computers sein, sondern es reicht aus, wenn der Computer auf eine passende externe Speichereinrichtung zugreifen kann. Eine Realisierung des erfindungsgemäßen Verfahrens in Form von Software hat den Vorteil, dass auch bestehende Analysesysteme relativ einfach durch geeignete Updates entsprechend nachgerüstet werden können. Bei dem erfindungsgemäßen Analysesystem kann es sich insbesondere auch um eine Ansteuerungseinheit für die Scandaten selbst aufzeichnende Modalität handeln, welche die notwendigen Komponenten zur erfindungsgemäßen Bearbeitung der Scandaten aufweist.

Die Erfindung wird im Folgenden anhand von Ausführungsbeispielen unter Hinweis auf die beigefügten Zeichnungen näher erläutert. Es zeigen:
Fig. 1 eine perspektivische Darstellung eines Zahnmodells M mit Grenzlinien LS ,LP und einem aktiven Anpassungsbereichen AA bzw. zwei inaktiven Anpassungsbereichen AI,
Fig. 2 eine perspektivische Darstellung eines Zahnmodells M mit anatomischen Landmarken L1, L2, L3, L4, L5 und Zahnachsen AX, AY,
Fig. 3 eine zweidimensionale schematische Darstellung von Scandaten D von Zähnen T13 bis T18 mit individualisierten Zahnmodellen M,
Fig. 4 eine zweidimensionale schematische Darstellung von Scandaten D von Zähnen T13 bis T18 mit Zahnrestaurationen R und ermittelten Präparationstypen (Inlay, Krone, Brücke, Zahn),
Fig. 5 eine perspektivische Darstellung von Scandaten D eines Oberkiefers mit segmentierten Zähnen T17 bis T27 und ermittelten Segmentierungslinien LS,
Fig. 6 eine perspektivische Darstellung von Scandaten D eines Oberkiefers mit segmentierten Zähnen T17 bis T27 und segmentiertem Zahnfleisch G,
Fig. 7 eine perspektivische Darstellung von Scandaten D eines Teil-Oberkiefers mit segmentierten Zähnen T35 bis T37 und ermittelten Segmentierungslinien LS und einer ermittelten Präparationslinie LP,
Fig. 8 eine perspektivische Darstellung von Scandaten D eines Teil-Oberkiefers mit segmentierten Zähnen T35 bis T37 und einer segmentierten Kavität C und segmentiertem Zahnfleisch G,
Fig. 9 eine perspektivische Darstellung von segmentierten Zähnen T17 bis T27 gemäß Fig. 5 und ermittelten Zahnachsen AY und ermittelten anatomischen Landmarken L4, L5,
Fig. 10 eine perspektivische Darstellung von segmentierten Zähnen T17 bis T27 gemäß Fig. 5 und einem ermittelten Winkel a14-15 zwischen zwei Zahnachsen, einer ermittelten Distanz d16-26 zwischen zwei anatomischen Landmarken und einer ermittelten Länge l17-27 eines anatomische Landmarken verbindenden Linienzugs,
Fig. 11 eine perspektivische Darstellung von Mittelwert-Zahnmodellen MM einer Modelldatenbank für Zahntypen Nr. 18 bis Nr. 48,
Fig. 12 eine Darstellung eines Zahnschemas, mit dem die vom Verfahren zu verwendenden Zahntypen definiert werden,
Fig. 13 eine Darstellung eines Zahnschemas, mit dem die vom Verfahren ermittelten Präparationstypen dargestellt werden,
Fig. 14 eine reduzierte Darstellung des Zahnschemas aus Fig. 13,
Fig. 15 eine perspektivische Darstellung von Scandaten D eines Kiefers, die zum Aufbau einer Modelldatenbank verwendet werden, wobei in der linken Bildhälfte der Oberkieferscan, und rechts der Unterkieferscan abgebildet ist,
Fig. 16 eine perspektivische Darstellung von Zahnmodellen M eines Zahntyps, die durch Anpassung an aus der Segmentierung von Kieferscans stammenden Scandaten eines Zahntyps konstruiert wurden,
Fig. 17 eine perspektivische Darstellung einer Aufstellung von Zahnmodellen M eines Zahntyps, definiert durch die gegenseitigen morphologischen Abweichungswerte, wobei morphologisch ähnliche Zahnmodelle M eine geringe räumliche Distanz bei der Aufstellung zueinander aufweisen,
Fig. 18 eine perspektivische Darstellung einer möglichen Sortierung von sieben Zahnmodellen M, bestehend aus einem Mittelwert-Zahnmodell MM und sechs Ziel-Zahnmodellen MT, einer Modelldatenbank mit sechs Teiltransformationen T1, T2, T3, T4, T5, T6,
Fig. 19 ein schematisch dargestelltes Ausführungsbeispiel eines erfindungsgemäßen Analysesystems.

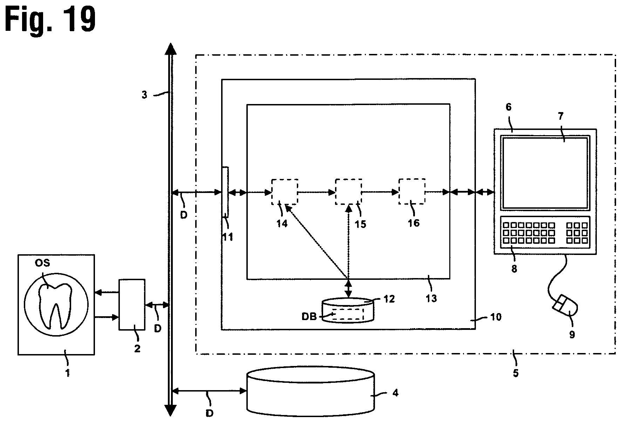

Das in Fig. 19 schematisch dargestellte Ausführungsbeispiel eines erfindungsgemäßen Analysesystems 5 besteht im Wesentlichen aus einem Computer 10 und einer daran angeschlossenen Konsole 6 o.Ä. mit einem Bildschirm 7, einer Tastatur 8 und einer Zeigeeinrichtung, hier einer Maus 9. Bei dem Computer 10 kann es sich um einen in üblicher Weise aufgebauten Computer, beispielsweise einen PC oder eine Workstation handeln, welcher auch zu sonstigen Datenauswertungen und/oder zur Steuerung von Bildaufnahmegeräten (Modalitäten) wie optischen Scannern, Computertomographen, Digitalen Volumentomographen, etc. eingesetzt werden kann. Wesentliche Komponenten innerhalb dieses Computers 10 sind u.a. ein zentraler Prozessor 13 und eine Schnittstelle 11, um Scandaten D oraler Strukturen OS zu empfangen, welche von einer Modalität 1, hier einem optischen Scanner, gemessen wurden.

In dem in Fig. 19 dargestellten Ausführungsbeispiel ist die Modalität 1 mit einer Steuereinrichtung 2 verbunden, welche wiederum mit einem Datenbus 3 verbunden ist, an dem auch das Analysesystem 5 angeschlossen ist. Außerdem sind an diesem Datenbus 3 ein Massenspeicher 4 zur Zwischenspeicherung oder dauerhaften Hinterlegung der von der Modalität 1 aufgezeichneten Scandaten D und/oder der von dem Analysesystem 5 weiter verarbeiteten Scandaten D angeschlossen. Selbstverständlich können am Datenbus 3 unter Bildung eines größeren Netzwerks noch andere Komponenten, beispielsweise weitere Modalitäten, Massenspeicher, Workstations, Ausgabegeräte wie Drucker, Filming-Stationen, Schleifeinheiten o.Ä. angeschlossen sein. Ebenso ist eine Verbindung mit einem externen Netz bzw. mit weiteren Analysesystemen möglich. Sämtliche Scandaten D werden dabei vorzugsweise zur Kommunikation im sog. STL-Standard (STL = Surface Tesselation Language) und/oder im DICOM-Standard (DICOM = Digital Imaging and Communication in Medicine) formatiert.

Die Ansteuerung der Modalität 1 erfolgt in üblicher Weise über die Steuereinrichtung 2, welche auch die Daten von der Modalität 1 akquiriert. Die Steuereinrichtung 2 kann zur Bedienung vor Ort eine eigene Konsole oder Ähnliches aufweisen, die hier jedoch nicht dargestellt ist. Es ist aber auch möglich, dass die Bedienung beispielsweise über den Datenbus mittels eines separaten Computers erfolgt, welche sich in der Nähe der Modalität befindet.

Zunächst werden in einem ersten Verfahrensschritt die Scandaten D ausgewählt und der Umfang der vom Verfahren zu verwendenden Zahntypen festgelegt. Die Scandaten D können beispielsweise unmittelbar von der Modalität 1 bzw. deren Steuereinrichtung 2 über den Datenbus 3 dem Computer 10 zugeführt werden. Es kann sich aber auch um Scandaten D handeln, die bereits vor einiger Zeit aufgenommen und in einem Massenspeicher 4 hinterlegt wurden. Bei dem im Folgenden beschriebenen Ausführungsbeispiel wird davon ausgegangen, dass es sich bei den zu analysierenden Scandaten D um optische und nicht um radiologische Scandaten D handelt. Eine geometrische Analyse von radiologischen Scandaten D erfolgt analog zu einer Analyse von optischen Scandaten D. Ein Unterschied besteht dabei in der Bestimmung von Zielstrukturen für die Zahnmodelle. Bei optischen Scandaten D bilden die gemessenen Oberflächen, oder Teile dieser Oberflächen, direkt die Zielstrukturen. Hingegen werden bei radiologischen Scandaten D in der Regel zuerst geeignete Oberflächen durch dem Fachmann bekannte Verfahren im Vorfeld der Analyse erzeugt. Beispielsweise kann man durch Schwellenwert- und/oder Konturanalyseverfahren aus CT/DVT-Scandatensätzen D die Oberflächen von Zähnen und/oder dem Zahnfleisch und/oder den Kieferknochen in einer triangulierten Form erhalten. Basierend auf diesen Oberflächendaten kann dann die geometrische Analyse der radiologischen Scandaten D analog zur geometrischen Analyse von optischen Scandaten D durchgeführt werden.

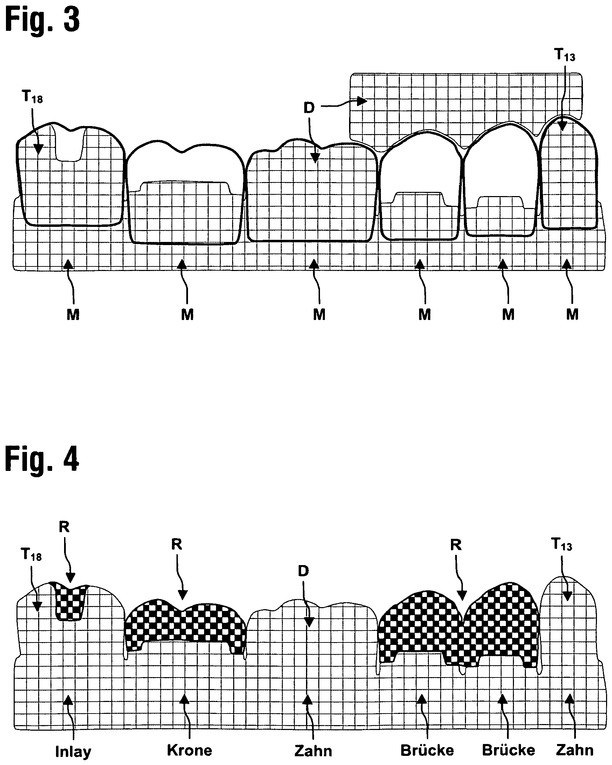

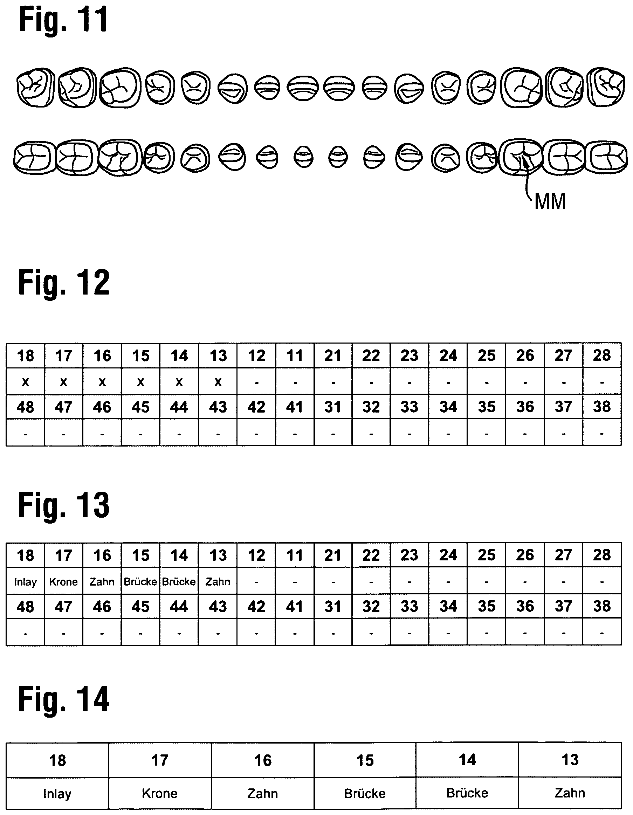

Der Umfang der vom Verfahren zu verwendenden Zahnmodelle wird vorzugsweise durch die Angabe einer Menge unterschiedlicher Zahntypen festgelegt. Optional kann der Bediener zusätzlich auch die Präparationstypen der ausgewählten Zahntypen angeben. Diese angegebenen Präparationstypen können dann zur Verfahrenskontrolle mit den ermittelten Präparationstypen verglichen werden oder aber auch in die Berechnung von Optimierungsteilwerten eingehen. Beispielsweise sollen bei Kronen-, bzw. Brückenpräparationen die Okklusionsflächen der dazugehörenden Zahnmodelle außerhalb von Zahn-, bzw. Restzahnsubstanz in den Scandaten D liegen. Wegen der besseren Anschaulichkeit für den Bediener können diese Angaben mittels einer grafischen Benutzeroberfläche und einem Zahnschema erfolgen, das beispielhaft in Fig. 12 dargestellt ist. Die dazugehörenden Mittelwert-Zahnmodelle MM der Zahntypen Nr. 18 bis Nr. 48 zeigt die Fig. 11. Vorzugsweise sollte der Umfang der zu verwendenden Zahntypen dem Umfang der Zahntypen der gescannten Zähne entsprechen. Im Folgenden wird davon ausgegangen, dass der Bediener mehrere Zahntypen mit den dazugehörenden Präparationstypen ausgewählt hat (siehe Fig. 13). In diesem Fall ist die Verwendung derjenigen Variante des erfindungsgemäßen Verfahrens sinnvoll, bei der Gruppen von Zahnmodellen individualisiert werden. Man kann aber auch die Präparationstypen mittels eines reduzierten Zahnschemas (siehe Fig. 14) oder grafisch mit den Scandaten D darstellen (siehe Fig. 4). Diese Figur zeigt auch die aus den individualisierten Zahnmodellen und Scandaten D ermittelten Zahnrestaurationen R.

Die Auswahl der Zahnmodelle aus einer Modelldatenbank DB kann mittels einer Auswahleinheit 14 stattfinden, welche hier in Form eines Softwaremoduls auf dem Prozessor 13 des Computers 10 realisiert ist (siehe Fig. 19). Die Modelldatenbank DB ist beispielsweise auf einem Speicher 12 des Computers 10 hinterlegt, auf den auch die Auswahleinheit 14 Zugriff hat, bzw. die Auswahleinheit 14 kann Auswahlvorgaben festlegen, die von nachfolgenden Arbeitsmodulen bei der Modellindividualisierung berücksichtigt werden.

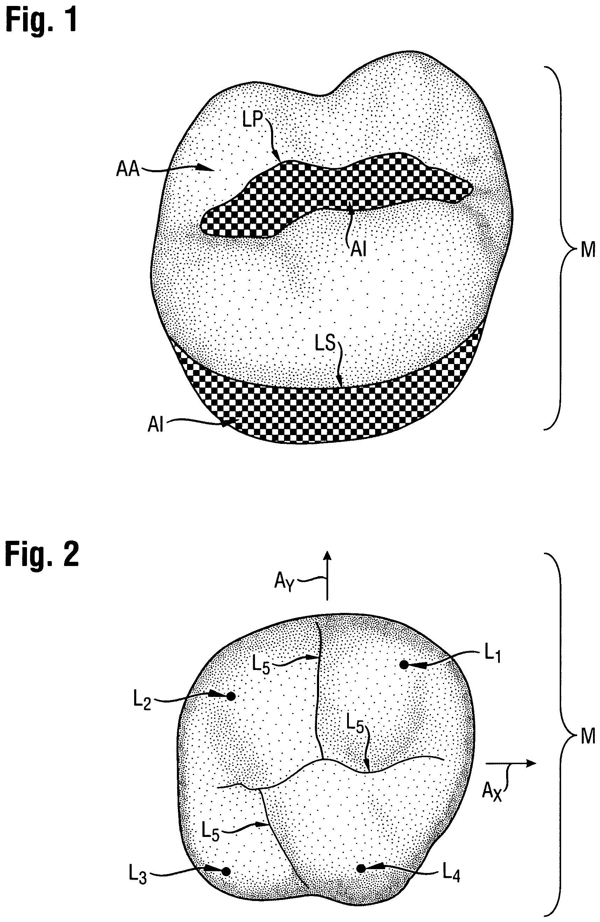

Ein Ausführungsbeispiel für ein 16-Zahnmodell M zeigt Fig. 1. Das Oberflächen-Zahnmodell M enthält eine Segmentierungslinie LS und eine Präparationslinie LP. Diese beiden Grenzlinien LS, LP definieren einen aktiven Anpassungsbereich AA und zwei inaktive Anpassungsbereiche AI. Die Grenzlinien LS, LP liegen bei diesem Ausführungsbeispiel auf der Oberfläche des Zahnmodells M. Da bei dem vorliegenden Ausführungsbeispiel von optischen Scandaten ausgegangen wird, ist die Verwendung von Oberflächenmodellen für die geometrische Analyse von Vorteil. Außerdem enthält das in Figur 1 gezeigte Zahnmodell M keine Teilmodelle, wie beispielsweise ein Implantat-Teilmodell. Die Parametrisierung der Grenzlinien LS, LP kann über aus der Computergrafik bekannte Texturkoordinaten des Zahnmodells M erfolgen. Dazu können jedem Vertex des Zahnmodells M zweidimensionale Texturkoordinaten zugeordnet werden. Die Vertices der Grenzlinien LS, LP ergeben sich dann aus einer Menge von zweidimensionalen Texturkoordinaten. Diese Texturkoordinaten können bei diesem Ausführungsbeispiel als Linienparameter des Zahnmodells M verwendet werden.

Die Bestimmung von individualisierten Zahnmodellen für die gewünschten Zahntypen erfolgt bei dem beschriebenen Ausführungsbeispiel durch die Lösung einer Optimierungsaufgabe, wobei beginnend mit Start-Zahnmodellen der gewünschten Zahntypen ein Optimierungsverfahren durchgeführt wird. Bei der Individualisierung der Zahnmodelle werden zuerst die Start-Zahnmodelle für die gewünschten Zahntypen bestimmt. Vorzugsweise sind die Start-Zahnmodelle in der Modelldatenbank festgelegt und sind besonders bevorzugt Mittelwert-Zahnmodelle der gewünschten Zahntypen. Es können aber auch Start-Zahnmodelle verwendet werden, die sich durch eine vorherige Anwendung des erfindungsgemäßen Verfahrens aus den dazugehörenden individualisierten Zahnmodellen ergeben.

Nach der Auswahl der Start-Zahnmodelle sollten diese vor der Anpassung an die Scandaten relativ zu den Scandaten grob platziert werden, d.h. es wird eine Vorpositionierung der Start-Zahnmodelle vorgenommen. Bei radiologischen Scandaten (z.B. CT/DVT-Scandaten) sind in der Regel Richtungsbezeichnungen (anterior, posterior, etc.) des Datensatzes bekannt und das Aufnahmevolumen umfasst normalerweise den Mundraum mit angrenzenden Strukturen. Ähnlich ist die Situation bei optischen Kieferscans. Auch dort werden in der Regel Richtungskonventionen für die Scandaten eingehalten. Die Okklusionsflächen der Zähne zeigen in eine definierte Achsrichtung und die grob parabelförmigen Zahnbögen sind zu einer anderen definierten Achsrichtung hin geöffnet. Bei intraoralen optischen Scans ist die Situation geringfügig unterschiedlich. Auch hier zeigen die Okklusionsflächen der Zähne in eine definierte Achsrichtung und für die Enden des gemessenen Teils eines Zahnbogens sind Richtungsbezeichnungen (z.B. mesial, distal) bekannt. Aufgrund dieser impliziten Richtungsinformationen und der bekannten Abmessungen der Scandaten können die Start-Zahnmodelle zum Verfahrensstart relativ zu den Scandaten platziert werden.

Anschließend werden dann die Zahnmodelle durch Variation von Modellparametern an die Scandaten angepasst. Fig. 3 zeigt exemplarisch Scandaten D mit individualisierten Zahnmodellen M für typische Inlay-, Kronen- und Brückenpräparationen.

Die Individualisierung von Zahnmodellen wird vorzugsweise durch die Lösung einer Optimierungsaufgabe durchgeführt, bei der sich der Optimierungswert aus einer Anzahl von Optimierungsteilwerten ergibt, die gewünschten Individualisierungskriterien entsprechen, von denen einige nachfolgend noch einmal erläutert werden. Die einzelnen Optimierungsteilwerte werden nach deren Berechnung zu einem einzigen Optimierungswert zusammengefasst. Vorzugsweise geschieht dies durch die Berechnung einer gewichteten Summe der Optimierungsteilwerte. Die Gewichtungsfaktoren ermöglichen dabei eine Steuerung des Einflusses der einzelnen Individualisierungskriterien. So kann beispielsweise ein hoher Gewichtungsfaktor für die Anpassung der Zahnmodelloberflächen an die Zähne, bzw. Restzahnsubstanzen in Kombination mit einem niedrigen Gewichtungsfaktor für die Anpassung der Grenzlinien an dazugehörende Zielstrukturen zu individualisierten Zahnmodellen führen, die sehr gut an die Zähne, bzw. Restzahnsubstanzen angepasst sind, dafür aber keine hundertprozentige Anpassung der Grenzlinien an die dazugehörenden Zielstrukturen aufweisen. Vorzugsweise werden die Gewichtungsfaktoren so gewählt, dass alle Individualisierungskriterien gleichermaßen gut eingehalten werden. Die Lösung der im Allgemeinen nichtlinearen Optimierungsaufgabe kann durch dem Fachmann bekannte Verfahren erfolgen. Eine einfache Möglichkeit ist die Bestimmung des minimalen Optimierungswertes durch Gradienten-Abstiegsverfahren.

Ein wichtiger Optimierungsteilwert beschreibt die Anpassung eines Zahnmodells an die dazugehörende Zahn- und/oder Restzahnsubstanz in den Scandaten. Die Bestimmung einer dazugehörenden Zielstruktur hängt vor allem von der Aufnahmemodalität der Scandaten ab. Bei dem dargestellten Ausführungsbeispiel werden optische Scandaten verwendet und die gemessene Oberfläche wird direkt als Zielstruktur verwendet. Die Berechnung des Optimierungsteilwerts erfolgt vorzugsweise durch die Bildung der Summe von Quadraten von Entfernungswerten der Zahnmodell-Vertices des Zahnmodells zur Zielstruktur. Analog wird auch die Berechnung des Optimierungsteilwerts für die Anpassung der Grenzlinien eines Zahnmodells an Strukturen in den Scandaten mit entsprechenden Flächenkrümmungen durchgeführt. Als Zielstruktur für die Segmentierungslinien können beispielsweise Scanelemente verwendet werden, deren konkave Oberflächenkrümmungen über einem Schwellenwert liegen. Eine Zielstruktur für Präparationslinien enthält beispielsweise Scanelemente mit konvexen Oberflächenkrümmungen über einem dazugehörenden Schwellenwert. Im Rahmen der erfindungsgemäß unterschiedlichen Gewichtung von aktiven und inaktiven Bereichen können dabei die Vertices der aktiven Anpassungsbereiche stärker als die der inaktiven Anpassungsbereiche gewichtet werden. Insbesondere kann man auch nur die Vertices der aktiven Anpassungsbereiche in die Berechnung des Optimierungsteilwertes eingehen lassen, d.h. die Vertices der inaktiven Anpassungsbereiche werden mit Null gewichtet. Als Entfernungswerte können die minimalen Entfernungen der Zahnmodell-Vertices zur Zielstruktur, aber auch die Entfernungen entlang der Flächennormalen der Zahnmodell-Vertices zur Zielstruktur verwendet werden.

Ein weiterer wichtiger Optimierungsteilwert beschreibt bei dem vorliegenden Ausführungsbeispiel den Kontakt eines Zahnmodells zur Gegenbezahnung. Dabei wird die Gegenbezahnung direkt oder indirekt durch ein Bissregistrat vermessen. In beiden Fällen erhält man so genannte Antagonisten-Daten. Zur Beschreibung der Kontaktsituation eines Zahnmodells zu einem dazugehörenden Antagonisten wird die Verwendung von vorzeichenbehafteten Entfernungswerten vorgeschlagen. Ein negatives Vorzeichen für einen Entfernungswert eines Zahnmodell-Vertex ist dann gegeben, wenn er innerhalb des Antagonisten liegt, ansonsten erhält er ein positives Vorzeichen. Bestimmt man nun das Minimum der Entfernungswerte, so erhält man für ein Zahnmodell einen einzigen vorzeichenbehafteten Kontakt-Enfernungswert. Ist er positiv, so liegt keine Durchdringung vor, ist er Null, so existieren ein oder mehrere Kontaktpunkte, ist er negativ, so liegt eine Durchdringung vor. Der Optimierungsteilwert für den Kontakt eines Zahnmodells zur Gegenbezahnung ist dann vorzugsweise das Quadrat des Kontakt-Entfernungswerts.

Eine Kopplung von Zahnmodellen wird vorzugsweise durch Optimierungsteilwerte berücksichtigt, welche die Kontakte und/oder die räumlichen Relationen der Lagen und/oder die räumlichen Relationen der Formen der gekoppelten Zahnmodelle beschreiben. Bei dem vorliegenden Ausführungsbeispiel wurden die Zahntypen Nr. 13 bis Nr. 18 vom Bediener ausgewählt (siehe Fig. 13). Die dazugehörenden sechs benachbarten Zahnmodelle können dann eine Kontakt-Kopplungsgruppe für den Kontakt von Zahnmodellen bilden. Vollkommen analog zu der vorgeschlagenen Berechnungsvorschrift zur Beschreibung des Kontaktes zur Gegenbezahnung kann dann ein Optimierungsteilwert für den Kontakt der Zahnmodelle untereinander berechnet werden. Dazu werden für die sechs Zahnmodelle vorzugsweise fünf Kontakt-Entfernungswerte berechnet und als Kontakt-Optimierungsteilwert wird dann die Summe der Quadrate dieser Kontakt-Entfernungswerte gebildet.

Der Umfang der Lagen-Kopplungsgruppe entspricht bei dem vorliegenden Ausführungsbeispiel dem Umfang der Kontakt-Kopplungsgruppe. Der dazugehörende Lagen-Optimierungsteilwert kann auf Basis der räumlichen Relationen von anatomischen Landmarken der gekoppelten Zahnmodelle berechnet werden. Die Bestimmung geeigneter Landmarken findet vorzugsweise im Rahmen des Aufbaus einer Modelldatenbank statt und kann interaktiv und/oder algorithmisch erfolgen. Eine vorgeschlagene Berechnungsart für den Optimierungsteilwert basiert darauf, dass anatomische Landmarken gekoppelter Zahnmodelle auf parametrisierten dreidimensionalen Kurven liegen sollen. Als Lagen-Optimierungsteilwert wird dann vorzugsweise die Summe der Quadrate der Entfernungswerte von den Landmarken zu den Kurven gebildet. Eine dem Fachmann bekannte Kurve ist beispielsweise die Speesche Kurve, auf der die Schneidekanten und Höcker der oberen Zähne liegen sollen.

Ein weiterer Kopplungs-Optimierungsteilwert beschreibt die räumlichen Relationen der Formen gekoppelter Zahnmodelle untereinander. Die dazugehörende Formen-Kopplungsgruppe wird bei dem vorliegenden Ausführungsbeispiel wieder aus den sechs Zahnmodellen Nr. 13 bis Nr. 18 gebildet. Zur Berechnung des Formen-Optimierungsteilwertes wird bei dem vorliegenden Ausführungsbeispiel davon ausgegangen, dass die verwendete Modelldatenbank auf der Basis einer repräsentativen Menge von Probanden-Scandaten aufgebaut wurde. Zu einem Zahnmodell der Modelldatenbank eines Zahntyps korrespondieren dann diejenigen Zahnmodelle der anderen Zahntypen, die vom gleichen Probanden stammen. Der Formen-Optimierungsteilwert für die Kopplung der Formen von Zahnmodellen ergibt sich dann vorzugsweise aus der Berechnung der Abweichungen von den Korrespondenzen der Zahnmodelle.

Dazu wird für jedes Zahnmodell der Formen-Kopplungsgruppe - im Folgenden Ausgangs-Zahnmodell genannt - zuerst ein Probanden-Zahnmodell aus der Modelldatenbank bestimmt. Die Zuordnung eines Ausgangs-Zahnmodells zu einem Probanden-Zahnmodell des gleichen Zahntyps kann über die Formparameter des Ausgangs-Zahnmodells erfolgen. Beim Aufbau der Modelldatenbank werden zu jedem Probanden-Zahnmodell diejenigen Formparameter der geometrischen Transformation des Mittelwert-Zahnmodells bestimmt, welche das Probanden-Zahnmodell optimal wiedergeben. Diese Formparameter werden im Folgenden Probanden-Formparameter genannt und vorzugsweise in der Modelldatenbank abgespeichert. Einem Ausgangs-Zahnmodell mit einem Ausgangs-Formparametersatz wird dann dasjenige Probanden-Zahnmodell zugeordnet, deren Probanden-Formparameter sich am geringsten von den Ausgangs-Formparametern des Ausgangs-Zahnmodells unterscheiden. Beispielsweise können dazu zuerst die Differenzen des Ausgangs-Formparametersatzes zu allen Probanden-Formparametern gebildet werden. Das Minimum des Quadrates dieser Differenzen definiert den gesuchten Probanden-Formparametersatz. Zu dem so ermittelten Probanden-Zahnmodell können dann die korrespondierenden Probanden-Zahnmodelle in der Modelldatenbank bestimmt werden. Dazu brauchen in der Modelldatenbank aber nur die Formparameter der Probanden-Zahnmodelle abgespeichert sein. Aus diesen können dann die ursprünglichen Probanden-Zahnmodelle konstruiert werden.

Danach findet eine Berechnung von morphologischen Abweichungswerten der bestimmten korrespondierenden Probanden-Zahnmodelle zu den dazugehörenden Zahnmodellen der Formen-Kopplungsgruppe statt. Die Berechnung eines morphologischen Abweichungswerts kann einfach und robust über die Summe von Entfernungswert-Quadraten der Vertices von zwei zu vergleichenden Zahnmodellen erfolgen, nachdem die beiden Zahnmodelle durch Translation und Rotation möglichst optimal zur Deckung gebracht wurden. Der so bestimmte morphologische Abweichungswert wird im Folgenden als Korrespondenz-Abweichungswert bezeichnet. Für jedes der sechs Zahnmodelle der beim vorliegenden Ausführungsbeispiel vorhandenen Formen-Kopplungsgruppe werden dann fünf Korrespondenz-Abweichungswerte bestimmt. Der Formen-Optimierungsteilwert für die Kopplung der Formen der Zahnmodelle ergibt sich dann als die Summe der Quadrate dieser Korrespondenz-Abweichungswerte.

Die bisher beschriebenen Optimierungsteilwerte können vollautomatisch, d.h. ohne die Einbeziehung von Bediener-Interaktionen berechnet werden. Die dazugehörenden Qualitätsteilwerte entscheiden bei dem vorliegenden Ausführungsbeispiel darüber, z.B. durch Unterschreitung eines vorgegebenen Schwellenwertes, ob der Bediener zur Markierung von Strukturen in den Scandaten aufgefordert wird, die bei einem nochmaligen Ablauf des Verfahrens zusätzlich mit in die Individualisierung von Zahnmodellen eingehen. D.h. bei einem nochmaligen Ablauf des Verfahrens werden neben den bisher einbezogenen Optimierungsteilwerten zusätzliche Optimierungsteilwerte auf der Basis von Bediener-Interaktionen berücksichtigt. Liegt beispielsweise der Qualitätswert eines Zahnmodells für die Anpassung an die Zahn- oder Restzahnsubstanz unter einem Schwellenwert, so wird der Bediener zur Markierung von unpräparierter Zahnoberfläche oder Restzahnsubstanz aufgefordert. Da bei dem vorliegenden Ausführungsbeispiel die Präparationstypen bekannt sind, wird der Bediener beispielsweise zur Markierung von unpräparierter Zahnsubstanz bei Präparationstypen aufgefordert, die das Vorhandensein von unpräparierter Zahnsubstanz implizieren. Dies sind beispielsweise die Präparationstypen Zahn, Inlay, Onlay etc..

Ein wichtiger Optimierungsteilwert auf der Basis von Bediener-Interaktion beschreibt die Anpassung eines Zahnmodells an die dazugehörende markierte unpräparierte Zahnoberfläche in den Scandaten. Für jedes markierte Scanelement wird vorzugsweise die minimale Entfernung zur dazugehörenden Zahnmodelloberfläche bestimmt und aus diesen Werten ein mittlerer Entfernungswert berechnet. Das Quadrat dieses Entfernungswertes kann als Optimierungsteilwert verwendet werden. Vorzugsweise werden in den Scandaten zuerst diejenigen unpräparierten Zahnoberflächen markiert, die zum ersten und/oder letzten Zahnmodell in der Modellgruppe gehören. Vollkommen analog können diese Berechnungen auf die Bestimmung von Optimierungsteilwerten angewendet werden, die Kontaktpunkte zur Gegenbezahnung und/oder zu Nachbarzähnen beschreiben.

Bei der Anpassung eines Zahnmodells an die dazugehörende markierte Restzahnsubstanz in den Scandaten ist es vorteilhaft mit vorzeichenbehafteten Entfernungswerten zu arbeiten. Zumindest ein Berechnungsverfahren für vorzeichenbehaftete Entfernungswerte wurde bereits weiter oben beschrieben. Ein negatives Vorzeichen zeigt an, dass ein markiertes Scanelement innerhalb des Zahnmodells liegt, ein positives Vorzeichen zeigt hingegen an, dass ein markiertes Scanelement außerhalb liegt. Für alle markierten Scanelemente werden diese vorzeichenbehafteten Entfernungswerte bestimmt und das Quadrat des Maximums dieser Entfernungswerte als Optimierungsteilwert verwendet.

Die Berechnung eines Optimierungsteilwerts für die Anpassung eines Zahnmodells an eine vom Bediener markierten Segmentierungslinie in den Scandaten - beispielsweise durch einen einzigen Mausklick - kann auch durch die Verwendung von Entfernungswerten erfolgen. Dabei werden in der Regel konkave Oberflächenstrukturen, die den Übergang von den Zähnen zum Zahnfleische bilden, als Teile von Segmentierungslinien markiert. Zur Berechnung des dazugehörenden Optimierungsteilwertes wird vorzugsweise für jedes markierte Scanelement die minimale Entfernung zur Segmentierungslinie des dazugehörenden Zahnmodells bestimmt. Das Quadrat des mittleren Entfernungswertes aller so bestimmten Entfernungen wird dann für den Optimierungsteilwert verwendet. Der Optimierungsteilwert für die Anpassung eines Zahnmodells an eine markierte Präparationslinie erfolgt vollkommen analog, wobei sich Präparationslinien im Gegensatz zu Segmentierungslinien durch konvexe Oberflächenstrukturen auszeichnen.

Ein weiterer Optimierungsteilwert beschreibt die Anpassung eines Zahnmodells an markierte anatomische Landmarken in den Scandaten. Zur Berechnung des dazugehörenden Optimierungsteilwerts werden in einer bevorzugten Variante die Entfernungen der markierten anatomischen Landmarken in den Scandaten zu den dazugehörenden Landmarken des Zahnmodells berechnet. Der Optimierungsteilwert ergibt sich dann über die Summe der Quadrate dieser Entfernungswerte. In der gleichen Weise kann auch ein Optimierungsteilwert für die Anpassung eines Zahnmodells an nichtnatürliche orale Strukturen berechnet werden. Beispielsweise kann ein Zahnmodell auch ein Implantat-Teilmodell enthalten. Dieses Implantat-Teilmodell enthält vorzugsweise Landmarken, die in den Scandaten markiert werden können. Aus den Entfernungen von korrespondierenden Implantat-Landmarken ergibt sich dann der dazugehörende Optimierungsteilwert.

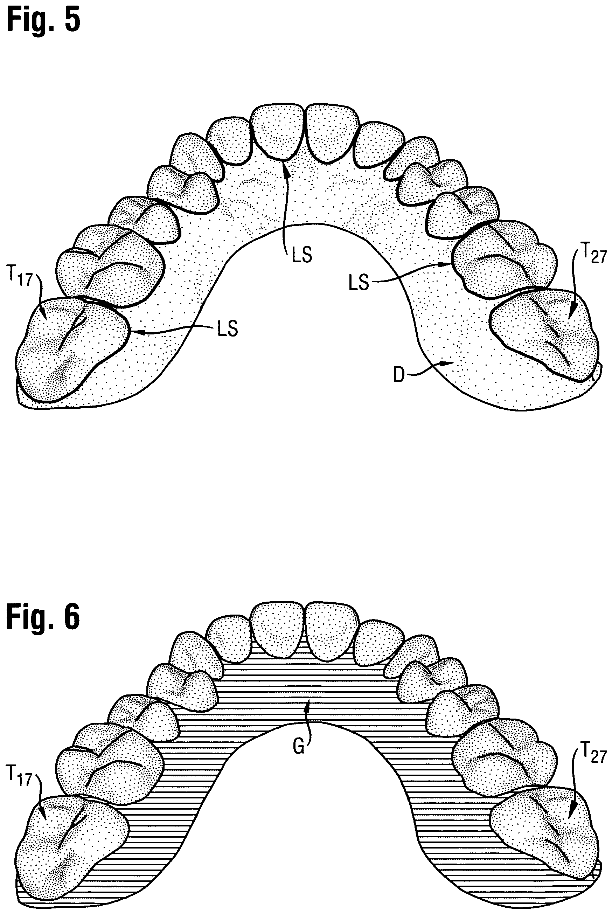

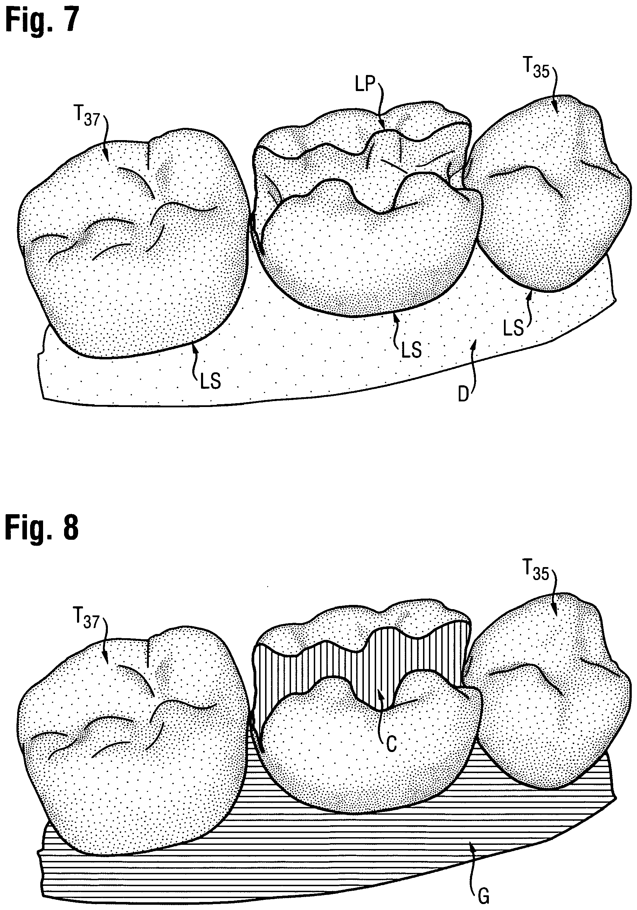

Zur Steigerung der Präzision der Verfahrensergebnisse kann man vor der weiteren geometrischen Analyse der Scandaten D eine Feinanpassung der individualisierten Zahnmodelle mit ihren individualisierten Grenzlinien LS, LP untereinander und/oder an die Scandaten D durchführen. Von Vorteil ist die Anwendung von Deformationstransformationen auf die individualisierten Zahnmodelle, welche eine Feinanpassung der Zahnmodelle untereinander sowie an die Zähne, Restzahnsubstanzen, Gegenbezahnung, Bissregistrate, Kontaktpunkte, etc. mit möglichst geringen Verschiebungswerten vornehmen und dabei keine Kanten oder Faltungen erzeugen. Diese Anpassungen können analog zur Individualisierung der Zahnmodelle durchgeführt werden, wobei als Optimierungsparameter jetzt die Parameter der Deformationstransformationen Verwendung finden. Die Berechnung des zu minimierenden Optimierungswertes aus Optimierungsteilwerten kann unverändert übernommen werden. Eine mögliche geometrische Transformation für die Feinanpassung eines Zahnmodells wird beispielsweise in der Promotionsarbeit "Individualisierung von digitalen anatomischen Modellen durch Computertomographie" (Tank M., 2002, Institut für Rechtsmedizin der Universität Heidelberg) beschrieben. Die Fig. 5 zeigt beispielsweise auf diese Weise fein angepasste Segmentierungslinien LS für einen Kieferscan der Zähne Nr. 17 bis Nr. 27. Fig. 7 zeigt hingegen beispielsweise fein angepasste Grenzlinien LS, LP für eine Inlaypräparation.

Die Berechnung der individualisierten Zahnmodelle findet innerhalb einer Individualisierungseinheit 15 statt, welche hier in Form eines Softwaremoduls auf dem Prozessor 13 des Computers 10 realisiert ist (siehe Fig. 19). Die Individualisierungseinheit 15 hat Zugriff auf die Modelldatenbank DB im Speicher 12 des Computers 10, um für ausgewählte Zahntypen die dazugehörenden Zahnmodelle mit ihren geometrischen Transformationen zu laden. Außerdem benötigt die Individualisierungseinheit 15 Zugriff auf weitere Informationen in der Modelldatenbank DB, beispielsweise auf die Probanden-Formparameter um Formen-Optimierungsteilwerte für Formen-Kopplungsgruppen zu berechnen.

Auf Basis der individualisierten Zahnmodelle mit ihren individualisierten Grenzlinien werden schließlich die Scandaten segmentiert. Die Fig. 6 zeigt beispielsweise einen segmentierten Kieferscan bei dem das segmentierte Zahnfleisch G schraffiert dargestellt ist. Fig. 8 zeigt hingegen segmentierte Scandaten D mit einer Inlaypräparation, bei der neben dem Zahnfleisch G auch die zur Inlaypräparation gehörenden Kavität C schraffiert dargestellt ist. Jedem Scanelement (beispielsweise Vertices bei optischen Scans, Voxel bei radiologischen Scans) wird bei der Segmentierung beispielsweise eine Zahnnummer oder eine Nummer, die extradentale Strukturen kennzeichnet, zugeordnet. Diese Nummern können farblich kodiert werden und ermöglichen mittels einer entsprechenden Einfärbung der Scandaten D eine schnelle visuelle Kontrolle der Segmentierungsergebnisse durch den Bediener. Dabei kann die Zuordnung von Zahnnummern zu Scanelementen durch unterschiedliche Verfahren erfolgen. Ein einfaches Verfahren bestimmt zuerst für ein Scanelement die minimalen Entfernungen zu jedem einzelnen individualisierten Zahnmodell, wobei ein negatives Vorzeichen für die Entfernung verwendet wird, wenn das Scanelement innerhalb des individualisierten Zahnmodells liegt. Anschließend wird die Zahnnummer des individualisierten Zahnmodells mit der geringsten Entfernung ermittelt und dem Scanelement zugeordnet. Liegt diese Entfernung über einem positiven Schwellenwert, dann wird eine definierte Nummer zur Kennzeichnung von extradentalen Strukturen für das Scanelement verwendet.

Kavitäten C zeichnen sich bei diesem Verfahren dadurch aus, dass die minimale Entfernung eines Scanelements zu einem dazugehörigen Zahnmodell unterhalb eines negativen Schwellenwertes liegt. Entsprechende Markierungen von Kavitäten C können zusätzlich zu den Zahnnummern den Scanelementen zugeordnet werden. Eine weitere Differenzierung der Ergebnisse kann dadurch vorgenommen werden, dass bei den Berechnungen anschließend nur Teile der individualisierten Zahnmodelle betrachtet werden. So können den Scanelementen weitere Markierungen hinzugefügt werden, die einzelne Teilmodelle oder Oberflächenregionen beschreiben, wie z.B. Wurzeln oder Höcker.

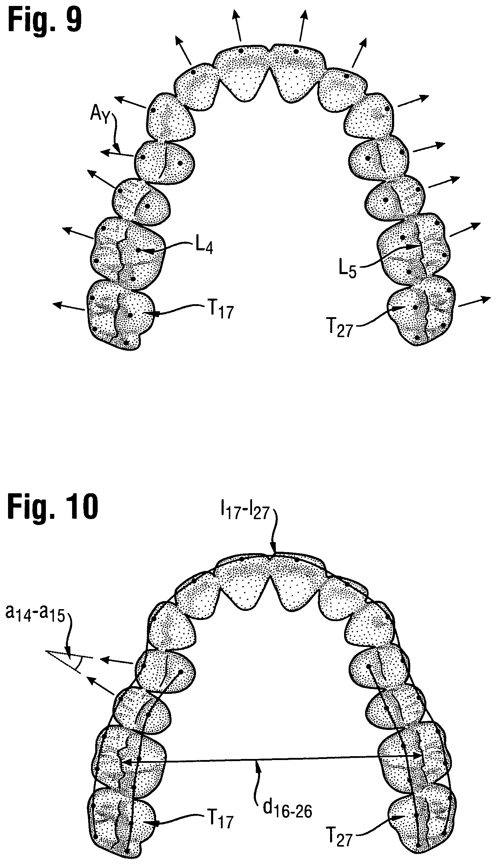

Die Bestimmung von Segmentierungslinien LS, Präparationslinien LP, anatomischen Landmarken L1, L2, L3, L4, L5, Zahnachsen AX, AY, Richtungsbezeichnungen und weiteren charakteristische geometrische Strukturen in den Scandaten D erfolgt durch die Übertragung der entsprechenden an den Zahnmodellen M markierten Strukturen (siehe Fig. 2) auf die Scandaten D. Fig. 9 zeigt eine perspektivische Darstellung von segmentierten Zähnen T17 bis T27 und auf diese Weise ermittelte Zahnachsen AY und ermittelte anatomische Landmarken L4, L5. Darüber hinaus können die Scandaten D auf Basis dieser individualisierten Strukturen vermessen werden. Die Fig. 10 zeigt eine perspektivische Darstellung von segmentierten Zähnen T17 bis T27 und einem ermittelten Winkel a 14-15 zwischen zwei Zahnachsen, einer ermittelten Distanz d16-26 zwischen zwei anatomischen Landmarken und einer ermittelten Länge l17-27 eines anatomische Landmarken verbindenden Linienzuges.

Diese weitere geometrische Analyse der Scandaten, auf Basis der individualisierten Zahnmodelle, findet innerhalb einer Analyseeinheit 16 statt, welche hier - der Individualisierungseinheit 15 nachgeordnet - ebenfalls in Form eines Softwaremoduls auf dem Prozessor 13 des Computers 10 realisiert ist.

Auf Basis der individualisierten Zahnmodellen und Scandaten lassen sich auch die Zahnrestaurationen R und/oder Präparationstypen (Inlay, Krone, Brücke, etc.) der gescannten Zähne bestimmen (siehe Fig. 4). Diese algorithmisch bestimmten Präparationstypen erlauben bei dem vorliegenden Ausführungsbeispiel eine Kontrolle der Verfahrensergebnisse, indem diese mit den durch den Bediener angegebenen Präparationstypen verglichen werden. Die Bestimmung von Zahnrestaurationen R und Präparationstypen kann auch innerhalb der Analyseeinheit 16 stattfinden.

Die Zahnrestaurationen R können aus den individualisierten Zahnmodellen M und Scandaten D durch Standardverfahren bestimmt werden, die dem Fachmann bekannt sind. Prinzipiell werden dazu die Präparationslinien der Scandaten D in geeigneter Weise möglichst stetig und glatt mit den individualisierten Zahnmodellen M verbunden und dann die Kavitäten als untere Begrenzungen hinzugefügt. Als Ergebnis erhält man für die Zahnrestaurationen R dreidimensionale Modelle von Schleifkörpern, die maschinell hergestellt werden können. In der Fig. 3 sind exemplarisch Scandaten D mit optimalen Zahnmodellen M für typische Inlay-, Kronen- und Brückenpräparationen dargestellt. Fig. 4 zeigt die dazugehörenden Zahnrestaurationen R.

Die Bestimmung von Präparationstypen geschieht vorzugsweise auf Basis der typischen charakteristischen geometrischen Formen der ermittelten virtuellen Zahnrestaurationen und der dazugehörenden Grenzlinien. So liegen beispielsweise bei einer Kronenpräparation die Segmentierungs- und Präparationslinie überall relativ eng zusammen und befinden sich ungefähr auf Zahnfleischniveau und der Okklusionsbereich des dazugehörenden individualisierten Zahnmodells ist vollständig präpariert. Bei einer typischen Veneerpräparation hingegen liegt die Präparationslinie oral auf Zahnfleischniveau und lingual kurz hinter der präparierten Schneidekante, also erheblich weiter okklusal. Mesial und distal reicht die Präparationslinie in der Regel nah an die Nachbarzähne heran, schließlich handelt es sich ja um eine vollständige Verblendschale im Frontzahnbereich. Das dazugehörende individualisierte Zahnmodell weist eine vollständige Präparation der Schneidekante und der oralen Anteile oberhalb des Zahnfleisches auf. Die größte Variation der Präparationslinien ist bei Inlaypräparationen vorhanden. Aufgrund der Restzahnsubstanz befindet sich - von okklusal betrachtet - zumindest ein Teil der Präparationslinie im Bereich des Zahnzentrums und dieser Teil weist auch einen relevanten Abstand zur Segmentierungslinie auf. Im Gegensatz zu den beiden anderen Präparationstypen ist der Okklusionsbereich des individualisierten Zahnmodells nicht vollständig präpariert, sondern mindestens ein Höcker ist noch vorhanden.

Diese qualitativen Beschreibungen zur Bestimmung des Präparationstyps lassen sich in mathematische Berechnungen fassen. Die Okklusionsbereiche der Seitenzähne und die Schneidekanten der Frontzähne können an den Zahnmodellen markiert werden. Durch die individualisierten Präparationslinien erhält man inaktive Modellbereiche, die präparierten Bereichen der gescannten Zähne entsprechen. Der Prozentsatz der präparierten Okklusionsfläche, bzw. der Schneidekante eines individualisierten Zahnmodells kann dann zur Fallunterscheidung herangezogen werden.

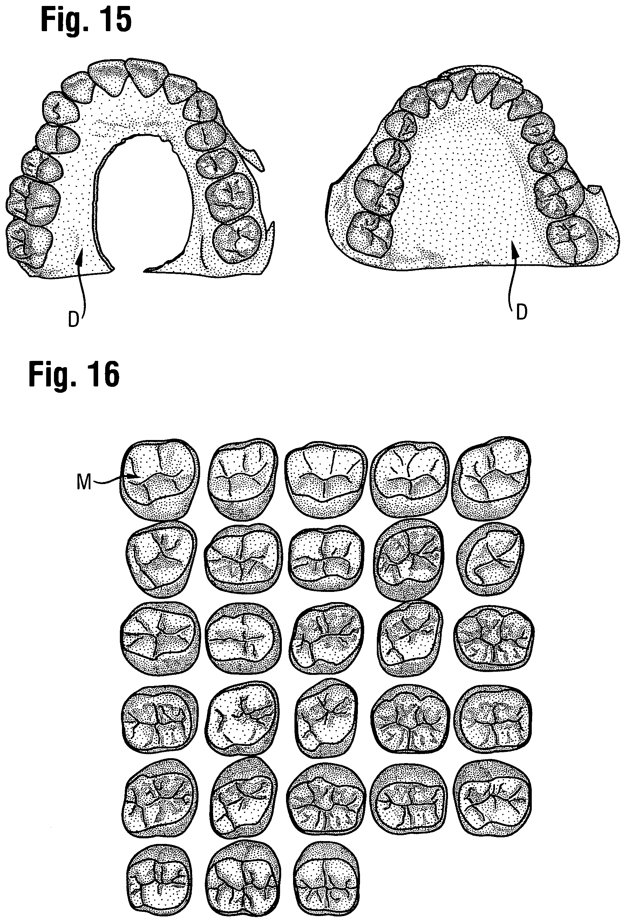

Ein Ausführungsbeispiel eines erfindungsgemäßen Verfahrens zum Aufbau von zumindest Teilen einer Modelldatenbank ist dadurch gekennzeichnet, dass eine Analyse einer repräsentativen Menge von Scandaten D defektfreier Ober- und Unterkiefer durchgeführt wird. Vorgeschlagen wird die Verwendung von Kiefer-Gipsabformungen, welche kostengünstig und präzise optisch eingescannt werden können. Fig. 15 zeigt auf diese Weise erzeugte optische Scandaten D. An dieser Stelle sei nochmals betont, dass der Datenbankaufbau aber auch durch eine Analyse von radiologischen Scandaten oder aber auch durch eine Analyse einer Kombination beider Datenarten stattfinden kann.

Für die Analyse der Formvariationen der Einzelzähne ist es vorteilhaft, die Scandaten D in einem ersten Schritt des Datenbankaufbaus zu segmentieren, wobei diese Segmentierung vorzugsweise interaktiv mit Hilfe einer grafischen Benutzeroberfläche durchgeführt werden kann. Als Ergebnis der Segmentierung von Kiefer-Scandaten D erhält man für jeden Zahntyp (hier Zahnnummer) eine Menge von Zahn-Scandatensätzen, welche zum Aufbau zumindest eines Teils einer Modelldatenbank verwendet werden kann.

Die Konstruktion von Zahnmodellen M eines Zahntyps findet im zweiten Schritt des Datenbankaufbaus statt. Je nach gewünschter räumlicher Auflösung wird für jeden Zahn-Scandatensatz ein Zahnmodell M konstruiert. Dazu kann man zuerst ein Zahnmodell M konstruieren, welches aus einer triangulierten Oberfläche in Form eines Quaders mit den Abmessungen des Zahn-Scandatensatzes besteht, wobei sich die Triangulierung einer gewünschten Auflösung durch sukzessive Unterteilung der Seitenflächen des Quaders ergibt. Danach findet eine Anpassung des initial konstruierten Zahnmodells M an den Zahn-Scandatensatz statt, beispielsweise durch die Minimierung der Summe der Quadrate der Entfernungswerte der Zahnmodell-Vertices zur Zahnoberfläche in dem Zahn-Scandatensatz. Fig. 16 zeigt auf diese Weise konstruierte Zahnmodelle M eines Zahntyps.

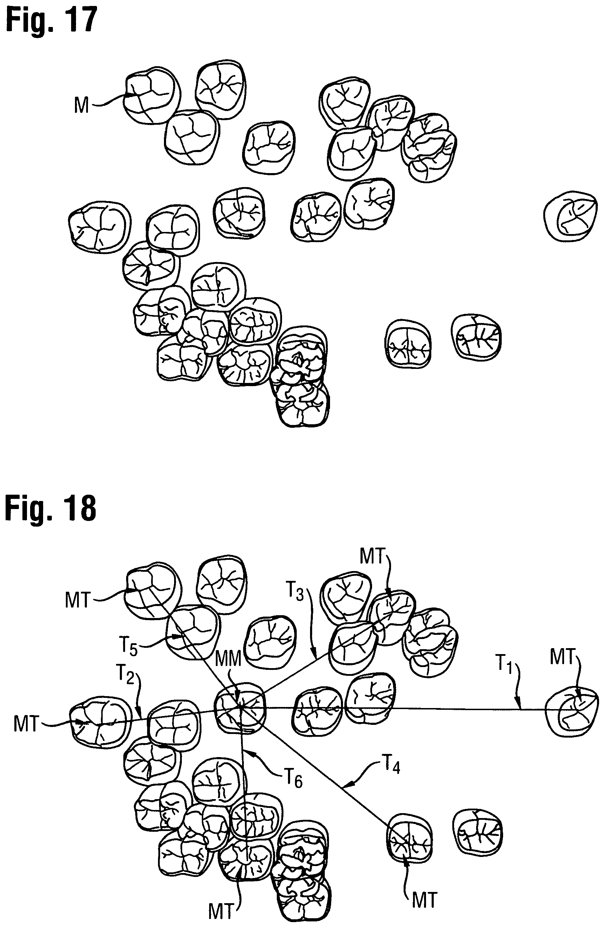

Anschließend findet im dritten Schritt des Datenbankaufbaus eine Analyse der morphologischen Abweichungen der Zahnmodelle M eines Zahntyps untereinander statt, indem für jedes mögliche Paar von Zahnmodellen M ein morphologischer Abweichungswert berechnet wird. Dazu werden die beiden Zahnmodelle M eines Paars vorzugsweise durch eine optimale Translation und Rotation derart zur Deckung gebracht, dass die Summe der Quadrate von Entfernungswerten der Zahnmodell-Vertices minimal ist. Als Entfernungswert eines Zahnmodell-Vertex des ersten Zahnmodells M des Paares kann der minimale Abstand des Vertex zur Oberfläche des zweiten Zahnmodells M des Paares verwendet werden. Der nach der Anpassung noch verbleibende mittlere Entfernungswert der Zahnmodell-Vertices kann als morphologischer Abweichungswert für das Zahnmodell-Paar verwendet werden. Eine Visualisierung der Ergebnisse erfolgt vorzugsweise durch eine zweidimensionale Aufstellung der Zahnmodelle M, wie in der Fig. 17 gezeigt. Diese Aufstellung ist dadurch definiert, dass die Entfernungen der Zahnmodelle M untereinander bis auf einen gemeinsamen Skalierungsfaktor möglichst den dazugehörenden morphologischen Abweichungswerten der Zahnmodelle M entsprechen.

Im vierten Schritt des Datenbankaufbaus wird für jeden Zahntyp ein Mittelwert-Zahnmodell MM bestimmt, indem vorzugsweise dasjenige Zahnmodell M ausgewählt wird, dessen Summe der Quadrate der morphologischen Abweichungswerte zu allen vorhandenen Zahnmodellen M des Zahntyps minimal ist. Dieses Zahnmodell M wird dann als Mittelwert-Zahnmodell MM des Zahntyps angesehen.

Im fünften Schritt des Datenbankaufbaus findet eine Sortierung der Zahnmodelle M eines Zahntyps auf Basis der berechneten morphologischen Abweichungswerte statt. Zum Aufbau einer Sortierung kann man die Sortierung mit dem Mittelwert-Zahnmodell MM beginnen und fügt iterativ weitere Zahnmodelle M des gleichen Zahntyps an. Das jeweils zuletzt angefügte Zahnmodell M wird dabei als aktuelles Zahnmodell M bezeichnet. Das Sortierkriterium kann dadurch definiert sein, dass immer dasjenige Zahnmodell M hinzugefügt wird, dessen Summe der Quadrate der morphologischen Abweichungswerte zu den bereits sortierten Zahmodellen M maximal ist.

Im abschließenden sechsten Schritt des Datenbankaufbaus wird noch eine parametrisierte geometrische Transformation zu dem Mittelwert-Zahnmodell MM hinzugefügt, vorzugsweise dadurch definiert, dass die Form des Mittelwert-Zahnmodells MM in zumindest ein in der Sortierung folgendes Ziel-Zahnmodelle MT kontinuierlich überführt werden kann. Diese geometrische Transformation setzt sich vorzugsweise aus geometrischen Teiltransformationen zusammen, wobei sich die Transformation vorzugsweise als Linearkombination aus den Teiltransformationen ergibt. Jede einzelne Teiltransformation transformiert das Mittelwert-Zahnmodell MM in ein Ziel-Zahnmodell MT. Die Fig. 18 zeigt ein Beispiel für eine Sortierung von sieben Zahnmodellen M, bestehend aus einem Mittelwert-Zahnmodell MM und sechs Ziel-Zahnmodellen MT, mit sechs dazugehörenden Teiltransformationen T1, T2, T3, T4. T5, T6. Diese sechs Ziel-Zahnmodelle MT sind die ersten Zahnmodelle, die beim Aufbau der Datenbank im fünften Schritt in der Sortierung dem Mittelwert-Zahnmodell MM nachfolgen, und somit die Zahnmodelle mit den größten morphologischen Abweichungen vom Mittelwert-Zahnmodell MM und den bereits vorher einsortierten Zahnmodellen (hier also den vorher zusortierten anderen Ziel-Zahnmodellen MT). Die Anzahl der Ziel-Zahnmodelle MT kann durch die Vorgabe eines Schwellenwertes für morphologische Abweichungswerte erfolgen. Dazu wird vorzugsweise die Anzahl der Ziel-Zahnmodelle MT solange erhöht und jedes mal die dazugehörende geometrische Transformation konstruiert, bis sich alle Zahnmodelle M des Zahntyps durch die Anwendung der konstruierten geometrischen Transformation bis auf den vorgegebenen Schwellenwert ergeben.

Der erste Schritt bei der Konstruktion der geometrischen Transformation besteht vorzugsweise in einer Unterteilung der Bounding-Box des Mittelwert-Zahnmodells MM in gleichgroße achsenparallele Zellen. Translationsvektoren der Zellenknoten definieren dann eine trilineare Transformation der Zahnmodell-Vertices. Dieser Ansatz wird im Detail in der Promotionsarbeit "Individualisierung von digitalen anatomischen Modellen durch Computertomographie" (Tank M., 2002, Institut für Rechtsmedizin der Universität Heidelberg) beschrieben und dort auch als Zellenverfahren bezeichnet. Als Ergebnis erhält man eine Transformation, bei der die Transformationsparameter hierarchisch nach ihrer Wichtigkeit geordnet sind. Die Transformationsparameter für ein Morphing zwischen dem Mittelwert-Zahnmodell MM und einem Ziel-Zahnmodell MT ergeben sich aus der Forderung, dass die Summe der Quadrate der Entfernungswerte des transformierten Mittelwert-Zahnmodells MM zu dem Ziel-Zahnmodell MT minimal ist und das lokale Flächeneigenschaften möglichst erhalten bleiben. Eine Linearkombination von auf dieser Weise erhaltenen Transformationsparametern ergibt morphologisch sinnvolle Variationen des Mittelwert-Zahnmodells MM. Die Formparameter des Mittelwert-Zahnmodells MM umfassen dann vorzugsweise die auf diese Weise definierten Linearfaktoren.

Neben dem Aufbau von zumindest Teilen einer Modelldatenbank auf der Basis von Scandaten natürlicher oraler Strukturen können aber auch Scandaten von künstlichen oralen Strukturen verwendet werden. Das Aufbauverfahren für die dazugehörende Modelldatenbank entspricht dem Aufbauverfahren für natürliche orale Strukturen und wird dabei vollkommen analog durchgeführt. Es kann dann der erste Schritt des Datenbankaufbaus entfallen. Der erste Schritt des Datenbankaufbaus, d.h. die Segmentierung der Scandaten, ist in der Regel nicht nötig, da die künstlichen oralen Strukturen (beispielsweise künstliche Zähne wie Prothesenzähne oder Zahnrohlinge) physisch separiert vorliegen und einzeln scannbar sind.

Es wird an dieser Stelle noch einmal ausdrücklich darauf hingewiesen, dass es sich bei den in den Figuren dargestellten Systemarchitekturen und Prozessen nur um Ausführungsbeispiele handelt, die vom Fachmann ohne weiteres im Detail verändert werden können. Insbesondere kann die Steuereinrichtung, sofern sie beispielsweise mit einer entsprechenden Konsole eingerichtet ist, auch alle entsprechenden Komponenten des Computers aufweisen, um dort unmittelbar die Messdatenverarbeitung nach dem erfindungsgemäßen Verfahren durchzuführen. In diesem Fall bildet folglich die Steuereinrichtung selbst das erfindungsgemäße Analysesystem, und ein weiterer bzw. separater Computer ist nicht erforderlich. Im Übrigen ist es auch nicht zwingend notwendig, dass die verschiedenen Komponenten eines erfindungsgemäßen Analysesystems auf einem Prozessor bzw. in einem Computer o.Ä. realisiert sind, sondern die verschiedenen Komponenten können auch auf mehrere Prozessoren bzw. untereinander vernetzten Computern verteilt sein. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließen die Begriffe "Vorrichtung", "Einrichtung", "Einheit", "Modul" etc. nicht aus, dass diese aus mehreren Komponenten bestehen, die gegebenenfalls auch räumlich verteilt sein können.

Es bietet sich im Übrigen an, bestehende Analysesysteme, in welchen bereits bekannte Nachverarbeitungsprozesse implementiert sind, mit einer erfindungsgemäßen Prozesssteuereinheit nachzurüsten, um auch diese Anlagen gemäß dem vorstehend beschriebenen erfindungsgemäßen Verfahren zu nutzen. In vielen Fällen reicht ggf. auch ein Update der Steuerungssoftware mit geeigneten Steuerungs-Softwaremodulen aus.

## Patentansprüche

1. Verfahren zur geometrischen Analyse von Scandaten (D) oraler Strukturen mittels eines Computers, bei dem
- entsprechend der zu analysierenden Scandaten (D) eine Anzahl parametrisierter Zahnmodelle (M) gewünschter Zahntypen ausgewählt wird, wobei die Parametrisierung anhand von Modellparametern erfolgt, welche Lage- und/oder Formparameter und Linienparameter umfassen und wobei jedes Zahnmodell (M) zumindest eine Grenzlinie (LS, LP) enthält, deren Verlauf durch die Linienparameter beschrieben wird und welche ein Zahnmodell (M) in zumindest einen aktiven (AA) und zumindest einen inaktiven Anpassungsbereich (AI) unterteilt,
- die Zahnmodelle (M) mit ihren Grenzlinien (LS, LP) zur Individualisierung an die Scandaten (D) angepasst werden, wobei die Individualisierung durch automatische Variation von Modellparametern durchgeführt wird und wobei die aktiven Anpassungsbereiche (AA) der Zahnmodelle (M) stärker gewichtet werden als die inaktiven Anpassungsbereiche (AI),
- auf Basis der individualisierten Zahnmodelle (M) mit ihren individualisierten Grenzlinien (LS, LP) automatisch die Scandaten (D) segmentiert werden und/oder zumindest eine Grenzlinie (LS, LP) in den Scandaten (D) bestimmt wird.

2. Verfahren nach Anspruch 1, **dadurch gekennzeichnet, dass** nach Individualisierung der Zahnmodelle (M) und vor einer weiteren geometrischen Analyse der Scandaten (D) eine Feinanpassung der individualisierten Zahnmodelle (M) mit ihren individualisierten Grenzlinien (LS, LP) untereinander und/oder an die Scandaten (D) stattfindet.

3. Verfahren nach einem der Ansprüche 1 bis 2, **dadurch gekennzeichnet, dass** die Grenzlinien (LS, LP) auf den Oberflächen der Zahnmodelle (M) liegen und/oder dass die Grenzlinien (LS, LP) einen parametrisierten Abstand zu den Oberflächen der Zahnmodelle (M) aufweisen und/oder das die Linienparameter so gewählt werden, dass die Grenzlinien (LS, LP) sich nicht schneiden und/oder einen Mindestabstand zueinander einhalten.

4. Verfahren nach einem der Ansprüche 1 bis 3, **dadurch gekennzeichnet, dass** die Grenzlinien (LS, LP) eines Zahnmodells (M) zumindest eine Segmentierungslinie (LS) und/oder zumindest eine Präparationslinie (LP) umfassen und wobei
- zur Individualisierung der Segmentierungslinien (LS) vorzugsweise eine Segmentierungslinien-Zielstruktur bestimmt wird, die aus Teilen von Scandaten (D) besteht und zumindest eine konkave Oberflächenstruktur umfasst,
- zur Individualisierung der Präparationslinien (LS) vorzugsweise eine Präparationslinien-Zielstruktur bestimmt wird, die aus Teilen von Scandaten (D) besteht und zumindest eine konvexe Oberflächenstruktur umfasst.

5. Verfahren nach einem der Ansprüche 1 bis 4, **dadurch gekennzeichnet, dass** die Anpassungsbereiche (AA, AI) auf den Zahnmodelloberflächen liegen und dass die aktiven Anpassungsbereiche (AA) bei optischen Scandaten (D) an unpräparierte Zahnoberflächen in den Scandaten (D) angepasst werden und bei radiologischen Scandaten (D) an unpräparierte Zahnoberflächen, die außerhalb des Zahnfleisches oder außerhalb des Kieferknochens liegen oder aus Zahnschmelz bestehen.

6. Verfahren nach einem der Ansprüche 1 bis 5, **dadurch gekennzeichnet, dass** für die Individualisierung von Zahnmodellen (M) ein Qualitätswert berechnet wird, der sich aus einzelnen Qualitätsteilwerten ergibt, die die Erfüllung von Individualisierungskriterien beschreiben und diese Qualitätsteilwerte darüber entscheiden, ob der Bediener zur Markierung von Strukturen in den Scandaten (D) aufgefordert wird, wobei die Markierungen bei einem nochmaligen Ablauf des Verfahrens mit in die Individualisierung von Zahnmodellen (M) eingehen.

7. Verfahren nach einem der Ansprüche 1 bis 6, **dadurch gekennzeichnet, dass** für ein Zahnmodell (M) die Gewichtung der Anpassungsbereiche (AA, AI) auf Basis der Qualitätsteilwerte für die Individualisierung der Grenzlinien (LS, LP) durchgeführt wird, wobei mit steigenden Qualitätsteilwerten für die Individualisierung der Grenzlinien (LS, LP) die aktiven Anpassungsbereiche (AA) bei der Anpassung an die Scandaten (D) stärker gewichtet werden.

8. Verfahren nach einem der Ansprüche 1 bis 7, **dadurch gekennzeichnet, dass** anatomische Landmarken (L1, L2, L3, L4, L5) und/oder Zahnachsen (AX, AY) und/oder Richtungsbezeichnungen und/oder Oberflächenregionen und/oder weitere charakteristische geometrische Strukturen an den Zahnmodellen (M) markiert und nach der Individualisierung auf die Scandaten (D) übertragen werden und/oder das auf Basis dieser individualisierten Strukturen eine geometrische Vermessung (d16-26, a14-15, 117-27) der Scandaten (D) vorgenommen wird.

9. Verfahren nach einem der Ansprüche 1 bis 8, **dadurch gekennzeichnet, dass** aus den individualisierten Zahnmodellen (M) und Scandaten (D) virtuelle Zahnrestaurationen (R) von präparierten Zähnen bestimmt werden und dass aus den charakteristischen geometrischen Formen der ermittelten virtuellen Zahnrestaurationen (R) und/oder zu den individualisierten Zahnmodellen (M) dazugehörenden individualisierten Grenzlinien (LS, LP) die Präparationstypen der zu den Zahnmodellen (M) dazugehörenden Zähne bestimmt werden und das optional sprachliche und/oder symbolische Bezeichnungen für diese ermittelten Präparationstypen mit einem Zahnschema und/oder mit einem reduzierten Zahnschema und/oder grafisch mit den Scandaten (D) dargestellt werden.

10. Verfahren nach einem der Ansprüche 1 bis 9, **dadurch gekennzeichnet, dass** die Individualisierung von Zahnmodellen (M) durch die Lösung einer Optimierungsaufgabe durchgeführt wird, bei der sich ein Optimierungswert vorzugsweise aus einer Anzahl von Optimierungsteilwerten ergibt, wobei zumindest einzelne der Optimierungsteilwerte besonders bevorzugt so gewählt sind, dass sie zumindest einem der folgenden Individualisierungskriterien entsprechen:
- die Anpassung von Zahnmodellen (M) an die Zähne und/oder Restzahnsubstanzen,
- die Anpassung von Zahnmodellen (M) an die Gegenbezahnung,
- die Anpassung von Zahnmodellen (M) an Bissregistrate,
- die Anpassung von Zahnmodellen (M) an nichtnatürliche orale Strukturen,
- die Anpassung von Grenzlinien (LS, LP) der Zahnmodelle (M) an Strukturen in den Scandaten (D) mit entsprechenden Oberflächenkrümmungen,
- die mechanische Stabilität der zu Zahnmodellen (M) gehörenden virtuellen Zahnrestaurationen (R),
- die ästhetische Wirkung der zu Zahnmodellen (M) gehörenden virtuellen Zahnrestaurationen (R),
- die Kontakte von Zahnmodellen (M),
- die räumlichen Relationen der Lagen von Zahnmodellen (M),
- die räumlichen Relationen der Formen von Zahnmodellen (M).

11. Verfahren nach Anspruch 10, **dadurch gekennzeichnet, dass** Strukturen in den Scandaten (D) markiert und Optimierungsteilwerte berechnet werden, die die Abweichungen der markierten Strukturen zu dazugehörenden Strukturen der Zahnmodelle (M) beschreiben, wobei bevorzugt zumindest eine Markierung eine der folgenden Strukturen beschreibt:
- unpräparierte Oberflächen von Zähnen,
- Restzahnsubstanzen von Zähnen,
- Segmentierungslinien von Zähnen,
- Präparationslinien von Zähnen,
- anatomischen Landmarken von Zähnen,
- nichtnatürliche orale Strukturen,
- Kontaktpunkte zu Nachbarzähnen,
- Kontaktpunkte zur Gegenbezahnung.

12. Verfahren zur Erzeugung einer Modelldatenbank (DB), die für verschiedene Zahntypen jeweils eine Anzahl von parametrisierten Zahnmodellen (M) enthält, zur Verwendung in einem Verfahren nach einem der Ansprüche 1 bis 11, wobei die Parametrisierung anhand von Modellparametern erfolgt, welche Lage- und/oder Formparameter und Linienparameter umfassen und wobei jedes Zahnmodell (M) zumindest eine parametrisierte Grenzlinie (LS, LP) enthält, deren Verlauf durch die Linienparameter beschrieben wird und welche ein Zahnmodell (M) in zumindest einen aktiven (AA) und zumindest einen inaktiven Anpassungsbereich (AI) unterteilt.

13. Verfahren nach Anspruch 12, **dadurch gekennzeichnet, dass** zumindest Teile der Modelldatenbank (DB) durch die Analyse einer Menge von optischen und/oder radiologischen Scandaten (D) oraler Strukturen aufgebaut werden, bei dem für einen gewünschten Zahntyp
- die Scandaten (D) optional segmentiert werden, um Scandaten (D) des gewünschten Zahntyps zu erhalten,
- Zahnmodelle (M) der Modelldatenbank (DB) durch eine Anpassung an die Scandaten (D) des gewünschten Zahntyps konstruiert werden,
- eine Analyse von morphologischen Abweichungen der Zahnmodelle (M) untereinander durchgeführt wird, indem für jedes mögliche Paar von Zahnmodellen (M) ein morphologischer Abweichungswert berechnet wird,
- ein Mittelwert-Zahnmodell (MM) auf Basis der morphologischen Abweichungswerte ausgewählt wird,
- eine Sortierung von Zahnmodellen (M) dadurch erzeugt wird, dass ausgehend vom Mittelwert-Zahnmodell (MM) die restlichen Zahnmodelle (M), auf Basis der morphologischen Abweichungswerte zu den bereits sortierten Zahnmodellen (M), an die Sortierung angefügt werden, wobei am Anfang der Sortierung diejenigen Zahnmodelle (M) stehen, die sich morphologisch stark unterscheiden,
- eine parametrisierte geometrische Transformation zu dem Mittelwert-Zahnmodell (MM) hinzugefügt wird, dadurch definiert, dass die Form des Mittelwert-Zahnmodells (M) in zumindest ein in der Sortierung folgendes Zahnmodell (MT) kontinuierlich überführt werden kann.

14. Computerprogrammprodukt, welches direkt in einen Speicher eines Computers ladbar ist, mit Programmcode-Mitteln, um alle Schritte eines Verfahrens nach einem der Ansprüche 1 bis 13 auszuführen, wenn das Programm auf dem Computer ausgeführt wird.

15. Computerimplementiertes Analysesystem (5) zur geometrischen Analyse von Scandaten (D) oraler Strukturen, mit
- einer Schnittstelle (11) zum Empfang der von einer Messeinrichtung gemessenen Scandaten (D),
- einer Speichereinrichtung (12) mit parametrisierten Zahnmodellen (M), wobei die Parametrisierung anhand von Modellparametern erfolgt, welche Lage- und/oder Formparameter und Linienparameter umfassen und wobei jedes Zahnmodell (M) zumindest eine parametrisierte Grenzlinie (LS, LP) enthält, deren Verlauf durch die Linienparameter beschrieben wird und welche ein Zahnmodell (M) in zumindest einen aktiven (AA) und zumindest einen inaktiven Anpassungsbereich (AI) unterteilt,
- einer Auswahleinheit (14), um den Umfang der vom Verfahren zu verwendenden Zahnmodelle (M) festzulegen,
- einer Individualisierungseinheit (15), durch die die Zahnmodelle (M) mit ihren Grenzlinien (LS, LP) zur Individualisierung an die Scandaten (D) anpasst werden, wobei diese Individualisierung durch Variation von Modellparametern durchgeführt wird und wobei die aktiven Anpassungsbereiche (AA) der Zahnmodelle (M) stärker gewichtet werden als die inaktiven Anpassungsbereiche (AI),
- und einer Analyseeinheit (16), durch die auf Basis der individualisierten Zahnmodelle (M) mit ihren individualisierten Grenzlinien (LS, LP) die Scandaten (D) segmentiert werden und/oder zumindest eine Grenzlinie (LS, LP) in den Scandaten (D) bestimmt wird.

## Claims

1. Method for the geometrical analysis of scan data (D) from oral structures by means of a computer, comprising the steps of
- selecting a number of parameterized tooth models (M) of desired tooth types according to the scan data (D) to be analyzed, whereby the parameterization is performed on the basis of model parameters comprising position parameters and/or shape parameters and line parameters, and whereby each tooth model (M) comprises at least one boundary line (LS, LP) whose path is defined by the line parameters and which divides a tooth model (M) in at least one active adjustment area (AA) and at least one inactive adjustment area (AI),
- adjusting the tooth models (M) and their boundary lines (LS, LP) to the scan data (D) for individualization, whereby the individualization is performed by automatic variation of model parameters, and whereby the active adjustment areas (AA) of the tooth models (M) are weighted more than the inactive adjustment areas (AI),
- automatically segmenting the scan data (D) on the basis of the individualized tooth models (M) and their individualized boundary lines (LS, LP) and/or determining at least one boundary line (LS, LP) in the scan data (D).

2. Method according to claim 1, **characterized in that**, after individualization of the tooth models (M) and prior to a further geometric analysis of the scan data, a fine adjustment is carried out of the individualized tooth models (M) with their individualized boundary lines (LS, LP) with respect to each other and/or to the scan data.

3. Method according to claim 1 or claim 2, **characterized in that** the boundary lines (LS, LP) lie on the surface of the tooth models (M) and/or that the boundary lines (LS, LP) comprise a parameterized distance from the surfaces of the tooth models (M) and/or that the line parameters are chosen such that the boundary lines (LS, LP) do not intersect and/or maintain a minimum distance from each other.

4. Method according to any of claims 1 to 3, **characterized in that** the boundary lines (LS, LP) of a tooth model (M) comprise at least one segmentation line (LS) and/or at least one preparation line (LP) and wherein
- a segmentation lines target structure, consisting of portions of scan data (D) and comprising at least one concave surface structure, is preferably determined for the individualization of the segmentation lines (LS);
- a preparation lines target structure, consisting of portions of scan data (D) and comprising at least one convex surface structure, is preferably determined for the individualization of the preparation lines (LP).

5. Method according to any of claims 1 to 4, **characterized in that** the adjustment areas (AA, AI) lie on the tooth model surfaces and that, in the case of optical scan data (D), the active adjustment areas (AA) are adjusted to unprepared tooth surfaces in the scan data (D) and, in the case of radiological scan data (D), to unprepared tooth surfaces which lie beyond the gum or beyond the jawbone or which comprise tooth enamel.

6. Method according to any of claims 1 to 5, **characterized in that** a quality value is computed for the individualization of tooth models (M), which quality value comprises individual quality partial values, which describe compliance with individualization criteria and which determine whether the user will be required to label structures in the scan data, which labels are used in the individualization of tooth models (M) in a repeated process cycle.

7. Method according to any of claims 1 to 6, **characterized in that**, for a tooth model (M), the weighting of the adjustment areas (AA, AI) is carried out on the basis of the quality partial values for the individualization of the boundary lines (LS, LP), whereby, with increasing quality partial values for the individualization of the boundary lines (LS, LP), the active adjustment areas (AA, AI) are weighted more in the adjustment to the scan data (D).

8. Method according to any of claims 1 to 7, **characterized in that** anatomic landmarks (L1, L2, L3, L4, L5) and/or tooth axes (AX, AY) and/or directional terms and/or surface regions and/or further characteristic geometric structures are labeled on the tooth models (M) and transferred to the scan data (D) after individualization, and/or a geometric measurement (d16-26, a14-15, l17-27) of the scan data (D) is performed on the basis of these individualized structures.

9. Method according to any of claims 1 to 8, **characterized in that** virtual tooth restorations (R) of prepared teeth are determined from the individualized tooth models (M) and scan data (D) and that the preparation types of the teeth associated with the tooth models (M) are determined from the characteristic geometric shapes of the determined virtual tooth restorations (R) and/or from the individualized boundary lines (LS, LP) belonging to the individualized tooth models (M) and that optional verbal and/or symbolic descriptors for these determined preparation types are displayed using a dental notation system and/or a reduced dental notation system and/or graphically using the scan data (D).

10. Method according to any of claims 1 to 9, **characterized in that** the individualization of tooth models (M) is performed by solving an optimization problem, in which an optimization value preferably results from a number of optimization partial values, whereby at least certain optimization partial values are particularly preferably chosen to satisfy at least one of the following individualization criteria:
- the adjustment of tooth models (M) to teeth and/or remaining tooth structure,
- the adjustment of tooth models (M) to opposing dentition,
- the adjustment of tooth models (M) to bite registrations,
- the adjustment of tooth models (M) to artificial oral structures,
- the adjustment of boundary lines (LS, LP) of the tooth models (M) to structures in the scan data (D) with corresponding surface curvatures,
- the mechanical stability of virtual tooth restorations (R) belonging to the tooth models (M),
- the aesthetic effect of virtual tooth restorations (R) belonging to the tooth models (M),
- the contacts of tooth models (M),
- the spatial relations of the positions of tooth models (M),
- the spatial relations of the shapes of tooth models (M).

11. Method according to claim 10, **characterized in that** structures are labeled in the scan data (D) and optimization partial values are computed that describe deviations of the labeled structures to corresponding structures of the tooth models (M), whereby preferably at least one label describes one of the following structures:
- unprepared surfaces of teeth,
- remaining tooth structure of teeth,
- segmentation lines of teeth,
- preparation lines of teeth,
- anatomic landmarks of teeth,
- artificial oral structures,
- contact points to neighboring teeth,
- contact points to opposing dentition.

12. Method of generating a model database (DB) comprising a number of parameterized tooth models (M) for each of a number of different tooth types, for use in the method according to any of claims 1 to 11, whereby parameterization is performed on the basis of model parameters, which comprise position parameters and/or shape parameters and line parameters, and whereby each tooth model (M) comprises at least one parameterized boundary line (LS, LP) whose path is described by line parameters and which divides a tooth model (M) in at least one active adjustment area (AA) and at least one inactive adjustment area (AI).

13. Method according to claim 12, **characterized in that** at least parts of the model database (DB) are built up by the analysis of a set of optical and/or radiological scan data (D) of oral structures, for which, for a desired tooth type
- the scan data (D) are optionally segmented in order to obtain scan data (D) of the desired tooth type,
- tooth models (M) of the model database (DB) are constructed by an adjustment to the scan data (D) of the desired tooth type,
- an analysis of morphological deviations is performed among the tooth models (M), in which a morphological difference value is computed for each possible pair of tooth models (M).
- a mean tooth model (MM) is chosen on the basis of morphological difference values,
- a sorting of tooth models (M) is generated by commencing with a mean tooth model (MM) and adding remaining tooth models (M) to the sort on the basis of morphological difference values to the already sorted tooth models (M), whereby tooth models (M) with significantly different morphology are at the beginning of the sort,
- a parameterized geometrical transformation is added to the mean tooth model (MM), defined **in that** the shape of the mean tooth model (MM) can be smoothly converted to at least one subsequent tooth model (MT) in the sort.

14. A computer program product, directly loadable in the memory of a computer, comprising program code means for carrying out all steps of a method according to any of claims 1 to 13 when said computer program product is run on the computer.

15. Computer-implemented analysis system (5) for the geometrical analysis of scan data (D) from oral structures, comprising
- an interface (11) for receiving scan data (D) measured by a measurement means,
- a memory means (12) comprising parameterized tooth models (M), whereby the parameterization is carried out on the basis of model parameters comprising position parameters and/or shape parameters and line parameters, and whereby each tooth model (M) comprises at least one parameterized boundary line (LS, LP), whose path is described by line parameters and which divides a tooth model (M) in at least one active adjustment area (AA) and at least one inactive adjustment area (AI),
- a selection unit (14) for defining the range of the tooth models (M) to be used by the procedure,
- an Individualization unit (15) in which the tooth models (M) with their boundary lines (LS, LP) are adjusted to the scan data (D) for individualization, whereby this Individualization is performed by variation of model parameters and whereby the active adjustment areas (AA) of the tooth models (M) are weighted more than the inactive adjustment areas (AI),
- and an analysis unit (16) in which the scan data (D) are segmented on the basis of the individualized tooth models (M) with their individualized boundary lines (LS, LP) and/or at least one boundary line (LS, LP) is determined in the scan data (D).

## Revendications

1. Procédé pour l'analyse géométrique de données de balayage (D) de structures orales au moyen d'un ordinateur, dans lequel
- conformément aux données de balayage à analyser (D), un certain nombre de modèles dentaires paramétrés (M) de types dentaires souhaités est sélectionné, dans lequel le paramétrage s'effectue à l'aide de paramètre de modèles qui comprennent des paramètres de position et/ou de forme et des paramètres de lignes, et dans lequel chaque modèle dentaire (M) contient au moins une ligne limite (LS, LP) dont le tracé est décrit par les paramètres de lignes et qui subdivise un modèle dentaire (M) en au moins une zone d'adaptation active (AA) et au moins une zone d'adaptation inactive (AI),
- les modèles dentaires (M) avec leurs lignes limites (LS, LP) étant adaptés pour l'individualisation aux données de balayage (D), dans lequel l'individualisation est réalisée par variation automatique de paramètres de modèles, et dans lequel les zones d'adaptation actives (AA) des modèles dentaires (M) sont plus fortement pondérées que les zones d'adaptation inactives (AI),
- sur la base des modèles dentaires (M) individualisés avec leurs lignes limites (LS, LP) individualisées, les données de balayage (D) étant automatiquement segmentées et/ou au moins une ligne limite (LS, LP) étant déterminée dans les données de balayage (D).

2. Procédé selon la revendication 1, **caractérisé en ce qu'**après l'individualisation des modèles dentaires (M) et avant une autre analyse géométrique des données de balayage (D), il y a une adaptation de précision des modèles dentaires (M) individualisés avec leurs lignes limites (LS, LP) individualisées entre eux et/ou aux données de balayage.

3. Procédé selon l'une des revendications 1 à 2, **caractérisé en ce que** les lignes limites (LS, LP) reposent sur les surfaces des modèles dentaires (M) et/ou **en ce que** les lignes limites (LS, LP) présentent une distance paramétrée par rapport aux surfaces des modèles dentaires (M) et/ou **en ce que** les paramètres des lignes sont sélectionnés de manière à ce que les lignes limites (LS, LP) ne se coupent pas et/ou conservent une distance minimale les unes par rapport aux autres.

4. Procédé selon l'une des revendications 1 à 3, **caractérisé en ce que** les lignes limites (LS, LP) d'un modèle dentaire (M) comprennent au moins une ligne de segmentation (LS) et/ou au moins une ligne de préparation (LP), et dans lequel
- pour l'individualisation des lignes de segmentation (LS), de préférence une structure cible de lignes de segmentation est déterminée, laquelle se compose de parties de données de balayage (D) et comprend au moins une structure de surface concave,
- pour l'individualisation des lignes de préparation (LP), de préférence une structure cible de lignes de préparation est déterminée, laquelle se compose de parties de données de balayage (D) et comprend au moins une structure de surface convexe.

5. Procédé selon l'une des revendications 1 à 4, **caractérisé en ce que** les zones d'adaptation (AA, AI) reposent sur les surfaces de modèle dentaire et **en ce que** les zones d'adaptation actives (AA), en cas de données de balayage (D) optiques, sont adaptées à des surfaces dentaires non préparées dans les données de balayage (D) et en cas de données de balayage (D) radiologiques, à des surfaces dentaires non préparées qui se situent en-dehors de la gencive ou en-dehors de l'os de la mâchoire ou se composent d'émail.

6. Procédé selon l'une des revendications 1 à 5, **caractérisé en ce que**, pour l'individualisation de modèles dentaires (M), on calcule une valeur de qualité qui résulte de valeurs partielles de qualité individuelles, lesquelles décrivent le respect de critères d'individualisation, et ces valeurs partielles de qualité déterminant si l'opérateur est appelé au marquage de structures dans les données de balayage (D), dans lequel, lors d'un nouveau déroulement du procédé, les marquages sont inclus dans l'individualisation de modèles dentaires (M).

7. Procédé selon l'une des revendications 1 à 6, **caractérisé en ce que**, pour un modèle dentaire (M), la pondération des zones d'adaptation (AA, AI) est réalisée sur la base des valeurs partielles de qualité pour l'individualisation des lignes limites (LS, LP), dans lequel, avec l'augmentation des valeurs partielles de qualité pour l'individualisation des lignes limites (LS, LP), les zones d'adaptation actives (AA) sont plus fortement pondérées lors de l'adaptation aux données de balayage (D).

8. Procédé selon l'une des revendications 1 à 7, **caractérisé en ce que** des repères anatomiques (L1, L2, L3, L4, L5) et/ou des axes dentaires (AX, AY) et/ou des indications de direction et/ou des zones de surface et/ou d'autres structures géométriques caractéristiques sont marqués sur les modèles dentaires (M) et sont transférés après l'individualisation aux données de balayage (D) et/ou **en ce que**, sur la base de ces structures individualisées, on procède à une mesure géométrique (d16-26, a14-15, l17-27) des données de balayage (D).

9. Procédé selon l'une des revendications 1 à 8, **caractérisé en ce qu'**à partir des modèles dentaires individualisés (M) et des données de balayage (D), on détermine des restaurations dentaires (R) virtuelles de dents préparées, et **en ce qu'**à partir des formes géométriques caractéristiques des restaurations dentaires virtuelles (R) déterminées et/ou des lignes limites (LS, LP) individualisées faisant partie des modèles dentaires (M) individualisés, on détermine les types de préparations des dents faisant partie des modèles dentaires (M) et **en ce qu'**optionnellement, des désignations linguistiques et/ou symboliques pour ces types de préparations déterminés sont représentées avec un schéma dentaire et/ou avec un schéma dentaire réduit et/ou graphiquement avec des données de balayage (D).

10. Procédé selon l'une des revendications 1 à 9, **caractérisé en ce que** l'individualisation de modèles dentaires (M) est réalisée par résolution d'une tâche d'optimisation où une valeur d'optimisation résulte de préférence d'un certain nombre de valeurs partielles d'optimisation, dans lequel au moins des valeurs partielles d'optimisation individuelles sont de façon particulièrement préférée sélectionnées de manière à ce qu'elles correspondent au moins à l'un des critères d'individualisation suivants :
- l'adaptation de modèles dentaires (M) aux dents et/ou substances dentaires résiduelles,
- l'adaptation de modèles dentaires (M) à la denture opposée,
- l'adaptation de modèles dentaires (M) à des enregistrements occlusaux,
- l'adaptation de modèles dentaires (M) à des structures orales non naturelles,
- l'adaptation de lignes limites (LS, LP) des modèles dentaires (M) à des structures dans les données de balayage (D) avec des courbures de surface correspondantes,
- la stabilité mécanique de restaurations dentaires (R) virtuelles appartenant à des modèles dentaires (M),
- l'effet esthétique des restaurations dentaires (R) virtuelles appartenant à des modèles dentaires (M),
- les contacts de modèles dentaires (M),
- les rapports spatiaux des positions de modèles dentaires (M),
- les rapports spatiaux des formes de modèles dentaires (M).

11. Procédé selon la revendication 10, **caractérisé en ce que** des structures dans les données de balayage (D) sont marquées et des valeurs partielles d'optimisation sont calculées, lesquelles décrivent les divergences des structures marquées par rapport à des structures associées des modèles dentaires (M), dans lequel de préférence au moins un marquage décrit l'une des structures suivantes :
- des surfaces non préparées de dents,
- des substances dentaires résiduelles de dents,
- des lignes de segmentation de dents,
- des lignes de préparation de dents,
- des repères anatomiques de dents,
- des structures orales non naturelles,
- des points de contact avec des dents voisines,
- des points de contact avec la denture opposée.

12. Procédé pour la production d'une base de données de modèles (DB), laquelle contient, pour différents types de dents, respectivement un certain nombre de modèles dentaires (M) paramétrés pour l'utilisation dans un procédé selon l'une des revendications 1 à 11, dans lequel le paramétrage s'effectue à l'aide de paramètres de modèles qui comprennent des paramètres de position et/ou de forme et des paramètres de lignes, et dans lequel chaque modèle dentaire (M) contient au moins une ligne limite (LS, LP) paramétrée dont le tracé est décrit par les paramètres de lignes et qui subdivise un modèle dentaire (M) en au moins une zone d'adaptation active (AA) et au moins une zone d'adaptation inactive (AI).

13. Procédé selon la revendication 12, **caractérisé en ce qu'**au moins des parties de la base de données de modèles (DB) sont constituées par l'analyse d'une quantité de données de balayage (D) optiques et/ou radiologiques de structures orales, avec lequel, pour un type de dent souhaité,
- les données de balayage (D) sont optionnellement segmentées pour obtenir des données de balayage (D) du type de dent souhaité,
- des modèles dentaires (M) de la base de données de modèles (DB) étant construits par une adaptation aux données de balayage (D) du type de dent souhaité,
- une analyse de divergences morphologiques entre les modèles dentaires (M) étant réalisée **en ce que**, pour chaque paire possible de modèles dentaires (M), on calcule une valeur de divergence morphologique,
- un modèle dentaire de valeur moyenne (MM) étant sélectionné sur la base des valeurs de divergence morphologique,
- un tri de modèles dentaires (M) étant généré **en ce que**, en partant du modèle dentaire de valeur moyenne (MM), sur la base des valeurs de divergence morphologique pour les modèles dentaires (M) déjà triés, les modèles dentaires (M) résiduels sont ajoutés au tri, dans lequel, au début du tri, se trouvent ceux des modèles dentaires (M) qui diffèrent fortement du point de vue morphologique,
- une transformation géométrique paramétrée étant ajoutée au modèle dentaire de valeur moyenne (MM), définie **en ce que** l'on peut faire passer de façon continue la forme du modèle dentaire de valeur moyenne (M) à au moins un modèle dentaire (MT) suivant dans le tri.

14. Produit de programme informatique pouvant être directement chargé dans une mémoire d'un ordinateur, avec des moyens de code de programme pour exécuter toutes les étapes d'un procédé selon l'une des revendications 1 à 13 lorsque le programme est exécuté sur l'ordinateur.

15. Système d'analyse mis en oeuvre par ordinateur (5) pour l'analyse géométrique de données de balayage (D) de structures orales, avec
- une interface (11) pour réceptionner les données de balayage (D) mesurées par un dispositif de mesure,
- un dispositif de mémoire (12) avec des modèles dentaires (M) paramétrés, dans lequel le paramétrage s'effectue à l'aide de paramètres de modèles comprenant des paramètres de position et/ou de forme et des paramètres de lignes, et dans lequel chaque modèle dentaire (M) contient au moins une ligne limite (LS, LP) paramétrée dont le tracé est décrit par les paramètres de lignes et qui subdivise un modèle dentaire (M) en au moins une zone d'adaptation active (AA) et au moins une zone d'adaptation inactive (AI),
- une unité de sélection (14) pour déterminer l'ampleur des modèles dentaires (M) à utiliser par le procédé,
- une unité d'individualisation (15) grâce à laquelle les modèles dentaires (M) sont adaptés avec leurs lignes limites (LS, LP) pour l'individualisation aux données de balayage (D), dans lequel cette individualisation est réalisée par variation de paramètres de modèles et dans lequel les zones d'adaptation actives (AA) des modèles dentaires (M) sont plus fortement pondérées que les zones d'adaptation inactives (AI),
- et une unité d'analyse (16) grâce à laquelle, sur la base des modèles dentaires (M) individualisés avec leurs lignes limite (LS, LP) individualisées, les données de balayage (D) sont segmentées et/ou au moins une ligne limite (LS, LP) est déterminée dans les données de balayage (D).
